(19) Europäisches Patentamt
European Patent Office
Office européen des brevets

(11) Veröffentlichungsnummer: **0 451 753 A1**

# (12) EUROPÄISCHE PATENTANMELDUNG

(21) Anmeldenummer: **91105514.3**

(22) Anmeldetag: **08.04.91**

(51) Int. Cl.[5]: **C07C 211/27**, C07F 15/00, A61K 31/28

(30) Priorität: **10.04.90 DE 4011520**

(43) Veröffentlichungstag der Anmeldung:
**16.10.91 Patentblatt 91/42**

(84) Benannte Vertragsstaaten:
**AT BE CH DE DK ES FR GB GR IT LI LU NL SE**

(71) Anmelder: **ASTA Pharma AG**
**Weismüllerstrasse 45**
**W-6000 Frankfurt am Main 1(DE)**

(72) Erfinder: **Brunner, Henri, Prof.**
**Eichendorffstrasse 14**
**W-8417 Lappersdorf(DE)**
Erfinder: **Hankofer, Peter, Dr.**
**Rybnikerstrasse 8**
**W-5000 Köln 80(DE)**
Erfinder: **Maiterth, Friedrich**
**Brunnthalerstrasse 6**
**W-8401 Hagelstadt(DE)**
Erfinder: **Engel, Jürgen, Prof.**
**Erlenweg 3**
**W-8755 Alzenau(DE)**
Erfinder: **Schumacher, Wolfgang, Dr.**
**Theodor-Heuss-Strasse 37**
**W-6070 Langen(DE)**
Erfinder: **Hilgard, Peter, Dr.**
**Kronenstrasse 11a**
**W-4800 Bielefeld(DE)**
Erfinder: **Voegeli, Rainer, Dr.**
**Bretonische Strasse 67**
**W-4800 Bielefeld(DE)**

(54) **Im Ethylenteil substituierte Phenyl-alkylethylendiamin-Platin (II oder IV)-Derivate und Phenylalkyl-ethylendiamine.**

(57) Antitumorwirksame Platin(II oder IV)-Komplexe der allgemeinen Formel

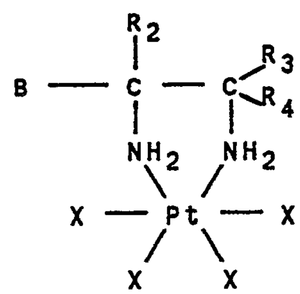

I

worin B einen Phenyl-$C_1$-$C_4$-alkylrest bedeutet, welcher gegebenenfalls im Phenylkern durch den Rest $R_1$ substituiert ist und $R_1$ Wasserstoff, Halogen, Trihalogenmethyl, $C_1$-$C_6$-Alkyl, Hydroxy, $C_1$-$C_6$-Alkoxy oder $C_2$-$C_6$-Alkanoyloxy ist oder worin B zusammen mit dem Strukturteil $H_2N$-$CR_2$ einen Tetrahydroisochinolinrest bildet, falls B Benzyl und $R_2$ Wasserstoff und der Benzylrest in 2-Stellung den $CH_2$-Rest enthält oder worin B zusammen mit dem Strukturteil -$CR_2$ einen Tetrahydronaphthylrest darstellt, bei dem gegebenenfalls eine $CH_2$-Gruppe durch Sauerstoff ersetzt ist, oder worin B zusammen mit dem Strukturteil -$CR_2$ einen Decahydronaphthylrest oder einen Indanylrest darstellt; $R_2$ Wasserstoff, $C_1$-$C_6$-Alkyl, Phenyl oder Phenyl-$C_1$-$C_4$-alkyl bedeutet, wobei der Phenylring dieser Gruppe $R_2$ auch durch Hydroxy, $C_1$-$C_4$-Alkoxy, $C_1$-$C_4$-Alkyl, $C_2$-$C_6$-Alkanoyloxy oder Halogen substituiert sein kann;
die Reste $R_3$ und $R_4$ gleich oder verschieden sind und Wasserstoff, $C_1$-$C_{12}$-Alkyl, $C_3$-$C_8$-Cycloalkyl, Phenyl,

EP 0 451 753 A1

welches gegebenenfalls durch $C_1$-$C_6$-Alkoxy substituiert ist bedeuten und X für das Äquivalent eines physiologisch verträglichen Anions steht oder X auch ein Wassermolekül sein kann, wobei im letzteren Falle die fehlende negative Ladung durch ein entsprechendes physiologisch verträgliches Säureanion abgesättigt wird, wobei im Falle von Platin(II)-Komplexen zwei der Gruppen X entfallen.

In der DOS 36 05 191 werden antitumorwirksame (1-Benzyl-ethylendiamin)-platin(II)-Komplexe der allgemeinen Formel

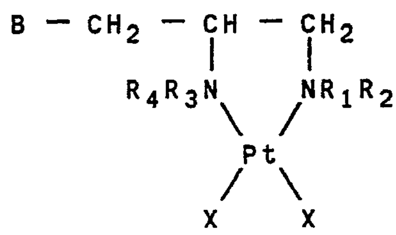

beschrieben, worin die Reste $R_1$, $R_2$, $R_3$ und $R_4$ gleich oder verschieden sind und Wasserstoff, eine $C_1$-$C_6$-Alkylgruppe, eine Benzylgruppe oder eine Phenylethylgruppe bedeuten, und B ein Thienylrest, ein Indolylrest, ein Imidazolylrest oder ein durch die Reste $R_5$, $R_6$ und $R_7$ substituierter Phenylrest ist und die Reste $R_5$, $R_6$ und $R_7$ gleich oder verschieden sind und Wasserstoff, Halogen, Trihalogenmethyl, $C_1$-$C_6$-Alkyl, Hydroxy, $C_1$-$C_6$-Alkoxy, Phenoxy, Benzyloxy, $C_1$-$C_6$-Alkansulfonyloxy, Carboxy, $C_1$-$C_6$-Carbalkoxy, Cyano, Aminocarbonyl, Aminocarbonyl, welches einen oder zwei $C_1$-$C_6$-Alkylreste enthält, $C_1$-$C_6$-Alkylcarbonyl, Nitro, Amino, $C_1$-$C_6$-Alkamino, Di-$C_1$-$C_6$-Alkylamino, $(C_1\text{-}C_6\text{-Alkyl})_3N^{\oplus}$, $C_1$-$C_6$-Alkanoyl-amino, $C_1$-$C_6$-Alkyl-$C_1$-$C_6$-alkanoyl-amino, $C_1$-$C_6$-Alkansulfonylamino, $C_1$-$C_6$-Alkyl-$C_1$-$C_6$-alkanoylamino, Aminosulfonyl, Aminosulfonyl, welches einen oder zwei $C_1$-$C_6$-Alkylreste enthält, $C_1$-$C_6$-Alkoxysulfonyl ($-SO_2$-O-$C_1$-$C_6$-Alkyl), Sulfo ($-SO_3H$) oder $C_1$-$C_6$-Alkansulfonyl bedeuten und zwei dieser Reste auch die Methylendioxygruppe sein können und X für das Äquivalent eines physiologisch verträglichen Anions steht.

Die folgenden Angaben betreffen bevorzugte Ausgestaltungen der Erfindung:
Die vorkommenden $C_1$-$C_6$-Alkylgruppen, $C_1$-$C_6$-Alkoxygruppen und die $C_1$-$C_6$-Alkanoyloxygruppen können gerade oder verzweigt sein. Die Alkyl- beziehungsweise Alkoxygruppen bestehen vorzugsweise aus 1 bis 4 C-Atomen, die Alkanoyloxygruppen vorzugsweise aus 2 bis 4 C-Atomen. Als Alkanoyloxygruppe kommt insbesondere die Acetoxygruppe in Frage. Als Halogensubstituenten kommen insbesondere Brom, Chlor und/oder Fluor in Frage. Im Falle der Phenyl-$C_1$-$C_4$-alkylgruppe besteht der Alkylteil vorzugsweise aus einem, zwei oder drei C-Atomen vorzugsweise handelt es sich um die Benzylgruppe (Phenylmethylgruppe) oder die 1-Phenylethylgruppe, wobei der Phenylteil gegebenenfalls jeweils wie angegeben substituiert sein kann, wobei sich Substituenten vorzugsweise in 4-Stellung befinden.

Besonders günstige Wirkung besitzen solche Verbindungen der Formel I, worin die Reste $R_3$ und $R_4$ Wasserstoff oder $C_1$-$C_4$-Alkyl, insbesondere Methyl und/oder $R_2$ eine $C_1$-$C_4$-Alkylgruppe (insbesondere Methyl oder Ethyl) eine Phenylgruppe oder eine durch Hydroxy (vorzugsweise p-Hydroxy) substituierte Phenylgruppe ist und B einen Benzylrest oder einen Phenylethyl-(1)-rest darstellt, wobei der Phenylring von B gegebenenfalls insbesondere durch Halogen (Cl) oder Hydroxy substituiert ist. Dieser Substituent (Halogen oder OH) befindet sich dabei vorzugsweise in der 4-Stellung.

Wenn B einen Phenyl-$C_1$-$C_4$-Alkylrest darstellt, ist dieser vorzugsweise geradkettig, die Phenylgruppe befindet sich bevorzugt in $\omega$-Stellung. Dasselbe gilt auch wenn $R_2$ Phenyl-$C_1$-$C_4$-alkyl bedeutet. Bei der $C_3$-$C_8$-Cycloalkylgruppe ($R_3$, $R_4$) handelt es sich vorzugsweise um die Cyclobutyl-, Cyclopentyl-, Cyclohexyl- oder Cycloheptylgruppe.
Falls es sich um Platin(IV)-Komplexe handelt, bedeuten 2 von den Gruppe X vorzugsweise jeweils 0H oder Halogen (Cl, Br) während für die beiden anderen X alle der hierfür angegebenen Bedeutungen in Frage kommen.

Falls B zusammen mit dem Strukturteil

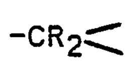

einen bicylischen Ring bildet, handelt es sich vorzugsweise um folgende Reste: Indanyl, vorzugsweise Indanyl-(2) (Formel siehe unten); Tetrahydronaphthyl, vorzugsweise Tetrahydronaphtyl-(1) oder Tetrahydronaphthyl-(2) (Formel siehe unten); 1-Oxa-tetrahydronaphthyl, vorzugsweise 1-Oxa-tetrahydronaphthyl-(4), Decahydronaphthyl, vorzugsweise Decahydronaphthyl-(1).

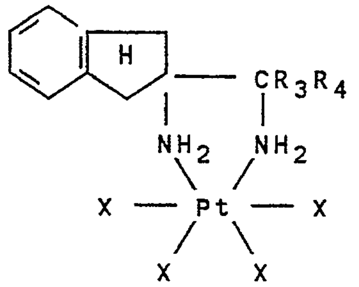

Indanyl-(2)-Komplex

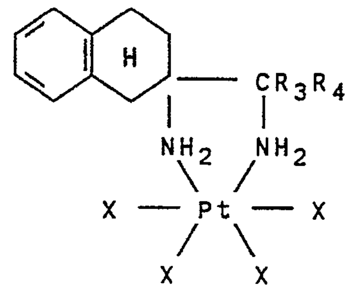

Tetrahydronaphthyl-(2)-Komplex

(Im Falle des Oxa-tetrahydronaphthylrestes ist in der rechten Formel eine $CH_2$-Gruppe des bicyclischen Ringes durch ein Sauerstoffatom ersetzt; im Falle des Decahydronaphthylrestes ist auch der Phenylteil des bicyclischen Restes vollständig hydriert).

Falls B zusammen mit dem Strukturteil

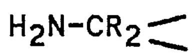

einen bicylischen Ring bildet (Tetrahydroisochinolinrest), handelt es sich um Verbindungen der folgenden Struktur:

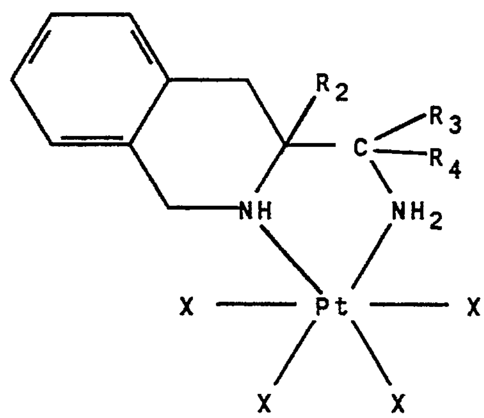

Die Reste X, die gleich oder verschieden sein können, stellen die bekannten und üblichen physiologisch verträglichen und pharmazeutisch verwendbaren Anionen ein- oder mehrwertiger Säuren oder auch das Hydroxyanion ($OH^-$) dar. Falls solche Säuren asymmetrische C-Atome haben, können diese als Racemate, als optisch reine Formen oder in Form der entsprechenden Diastereomere vorliegen. Insbesondere kommen beispielsweise die Anionen folgender Säuren in Frage: HBr, HCl, HJ, HF, $HNO_3$, $H_2SO_4$ - ($SO_4^{--}$); $H_3PO_4$ ($HPO_4^{--}$); $H_2CO_3$, ($CO_3^{--}$); HSCN, Kampfersulfonsäure, aliphatische oder aromatische Sulfonsäuren, beispielsweise $C_1$-$C_6$-Alkylsulfonsäuren (zum Beispiel Methansulfonsäure, Ethan-, Propan- oder Hexansulfonsäure), Benzol- oder Naphthalinsulfonsäure, die gegebenenfalls ein- oder zweifach durch Methylgruppen substituiert sind (Toluolsulfonsäure, insbesondere o- oder p-Toluolsulfonsäure), aliphatische $C_1$-$C_{20}$-Monocarbonsäuren insbesondere $C_1$-$C_{18}$-Monocarbonsäuren, die gegebenenfalls ein-, zwei- oder dreifach durch Halogenatome (insbesondere Cl, F) oder auch durch einen Phenylrest (in w-Stellung) substituiert sind (zum Beispiel Ameisensäure, Essigsäure, Propionsäure, Palmitinsäure, Stearinsäure, Chloressigsäure, Dichloressigsäure, Trifluoressigsäure, Trichloressigsäure, $\omega$-Phenlystearinsäure); aliphatische $C_2$-$C_{11}$-Dicarbonsäuren, die gegebenenfalls eine Doppelbindung enthalten (zum Beispiel Oxalsäure, Malonsäure, 2-Amino-Malonsäure, Malonsäure, welche in 2-Stellung durch eine Benzylgruppe oder eine oder zwei $C_1$-$C_4$-Alkylgruppen substituiert ist, Maleinsäure, Fumarsäure, Bernsteinsäure); aliphatische Monohydroxy-und Dihydroxy-monocarbonsäuren mit 2 bis 8, insbesondere 2 bis 6 Kohlenstoffatomen, wobei es sich vorzugsweise um $\alpha$-Monohydroxycarbonsäuren handelt wie Milchsäure, Glycerinsäure, Mandelsäure oder Glykolsäure; Di- und Tricarbonsäuren mit 3 bis 8 Kohlenstoffatomen, insbesondere 3 bis

6 Kohlenstoffatomen (zum Beispiel Äpfelsäure, Weinsäure, Malonsäure), die auch an einem C-Atom durch eine Hydroxygruppe und/oder gegebenenfalls eine $C_1$-$C_4$-Alkylgruppe substituiert sein können (Isozitronensäure, Zitronensäure); Phthalsäure, die gegebenenfalls durch eine Carboxygruppe (insbesondere in 4-Stellung) substituiert ist; Gluconsäure; Glucuronsäure; Azetidincarbonsäure; Quadratsäure (3,4-Dihydroxy-3-cyclobuten-1,2-dion); die natürlichen α-Aminosäuren (zum Beispiel L-Asparaginsäure); 1,1-Cyclobutandicarbonsäure; Organophosphorsäuren, wie Aldose- und Ketosephosphorsäuren (beispielsweise die entsprechenden Mono- und Diphosphorsäuren) zum Beispiel Aldose-6-phosphorsäuren wie D-oder L-Glucose-6-phosphorsäure, α-D-Glucose-1-phosphorsäure, D-Fructose-6-phosphorsäure, D-Galactose-6-phosphorsäure, D-Ribose-5-phosphorsäure, D-Fructose-1,6-diphosphorsäuren; Glycerinphosphorsäuren (wobei der Phosphorsäurerest an einem der endständigen oder an dem mittelständigen Glycerinsauerstoffatom gebunden ist) wie α-D,L-Glycerinphosphorsäure, ß-Glycerinphosphorsäure; N-Phosphono-acetyl-Asparaginsäure; Nitrilotris-methylphosphonsäure.

Vorzugsweise bedeutet X jeweils Chlor, Brom, Jod oder -SCN (Rhodanid) oder das Anion X leitet sich von einer Hydroxycarbonsäure der Struktur $R_5$-CH(OH)-$(CH_2)_n$-$CO_2H$ ab, wobei n die Werte 0, 1, 3 oder 4 annehmen kann und $R_5$ Wasserstoff, Halogen, Hydroxy, $C_2$-$C_6$-Alkanoyloxy, $C_1$-$C_6$-Alkoxy, $C_1$-$C_6$-Alkyl oder Phenyl, welches gegebenenfalls durch Halogen, Hydroxy, $C_1$-$C_6$-Alkoxy, $C_1$-$C_6$-Alkyl oder $C_1$-$C_6$-Alkanoyloxy substituiert ist, bedeutet.

Im Falle einer solchen Oxycarbonsäure hat der Komplexteil

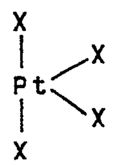

die folgende Struktur

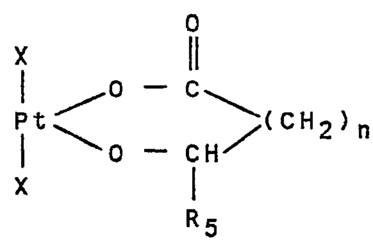

Zwei Gruppen X entfallen im Falle von Platin(II)-Komplexen oder sind vorzugsweise jeweils OH, Cl oder Br. Vorzugsweise leitet sich X von der Milchsäure, Mandelsäure oder der Glykolsäure (jeweils Racemat, D-Form, L-Form) ab.

Als Säuren für die Anionen X kommen weiterhin in Frage: Aromatische Carbonsäuren, die eine oder mehrere Carboxygruppen enthalten sowie außerdem noch eine oder mehrere (zum Beispiel eine, zwei, drei, vier oder fünf) $C_1$-$C_4$-Alkoxygruppen und/oder Hydroxygruppen (beispielsweise Salicylsäure). Falls sich mehrere Carboxygruppen an dem aromatischen Rest (zum Beispiel Benzolring) befinden, stehen mindestens 2 Carboxygruppen bevorzugt zueinander in Nachbarstellung. Falls der Benzolring zum Beispiel 4 oder 5 Carboxygruppen enthält, können Komplexe entstehen, die pro 1 Mol des Benzolcarbonsäureanions 2 Mol der Platinkomponente enthalten. 2 benachbarte Carboxygruppen neutralisieren jeweils 1 Mol der Platinkomponente, so daß zum Beispiel im Falle der Benzolpentacarbonsäure die 1- und 2-ständigen sowie die 4- und 5-ständigen Carboxygruppen jeweils 1 Mol der Platinkomponente absättigen (zusammen also 2 Mol), während die freie 3-ständige Carboxygruppe frei oder in der Salzform mit einem physiologisch verträglichen Kation (zum Beispiel Alkalikation, insbesondere Natriumkation) vorliegt. Dies gilt ganz allgemein, wenn die Anionen X noch zusätzliche Säurefunktionen besitzen, die nicht für die Absättigung des Platins gebraucht werden. Das Analoge gilt für den Fall der Benzolhexacarbonsäure, wobei hier gegebenenfalls 1 Mol dieser Säure 3 Mol der Platinkomponente absättigen kann.

Beispiel für solche Säuren sind: Benzolmonocarbonsäure, Benzoldicarbonsäuren, Benzoltricarbonsäuren (zum Beispiel Trimellithsäure), Benzoltetracarbonsäuren, Benzolpentacarbonsäure, Benzolhexacarbonsäure; Syringasäure, Orotsäure.

Ebenfalls kommen als Säuren, die die Anionen X bilden, Aminosäuren beziehungsweise Aminosäurederivate, deren basische Aminogruppe durch eine Säuregruppe geschützt ist, in Frage. Es handelt sich hierbei zum Beispiel um Aminosäuren der folgenden Struktur:

$$\begin{array}{c} R' - \underset{\displaystyle NH_2}{\underset{|}{CH}} - CO_2H \end{array}$$

worin R' Wasserstoff, einen Phenylrest, einen Indolyl-(3)-methylrest, Imidazolyl-(4)-methylrest, eine $C_1$-$C_{10}$-Alkylgruppe oder eine $C_1$-$C_{10}$-Alkylgruppe, die durch eine Hydroxygruppe, eine Aminogruppe, eine Carboxygruppe, eine $C_1$-$C_6$-Alkoxygruppe, eine Mercaptogruppe, eine $C_1$-$C_6$-Alkylthiogruppe, eine Phenylgruppe, eine Hydroxyphenylgruppe, eine $C_2$-$C_6$-Alkanoylaminogruppe oder eine $C_1$-$C_6$-Alkoxycarbonylgruppe substituiert ist, bedeutet.

Die basische Aminogruppe in 2-Stellung ist hierbei durch eine übliche Aminosäureschutzgruppe geschützt (acyliert), beispielsweise durch einen $C_2$-$C_6$-Alkanoylrest, den Benzoylrest oder den Butyloxycarbonylrest. Falls in der obigen Formel R' eine Alkylgruppe ist, handelt es sich vorzugsweise um eine $C_1$-$C_6$-Alkylgruppe,die zum Beispiel in 2-, 3-, 4-, 5- oder 6-Stellung (Zählung beginnt an der Verknüpfungsstelle des Alkylrestes mit dem Restmolekül) eine $C_2$-$C_6$-Alkanoylaminogruppe, einen Imidazolyl-(4)-methylrest oder einen Indolyl-(3)-methylrest enthält. Einzelbeispiele für solche Aminosäuren sind: Leucin (vorzugsweise D-oder L-Form), Valin (vorzugsweise D- oder L-Form), Phenylalanin (vorzugsweise D- oder L-Form), Phenylglycin (vorzugsweise D- oder L-Form), Alanin (vorzugsweise D-oder L-Form), Isoleucin (vorzugsweise D- oder L-Form), Asparagin (vorzugsweise D- oder L-Form), Lysin (vorzugsweise D- oder L-Form), Tryptophan (vorzugsweise D-oder L-Form, Tyrosin (vorzugsweise D-oder L-Form), Ornithin (vorzugsweise D- oder L-Form), Hydroxyprolin (D- oder L-Form).

Hierbei sind die basischen Aminogruppen durch eine übliche Acylaminoschutzgruppe blockiert, insbesondere durch die Acetylgruppe, Chloracetylgruppe, Benzoylgruppe oder die Butyloxycarbonylgruppe.

Gegebenenfalls können auch die entsprechenden Säureadditionssalze unter Verwendung physiologisch verträglicher Säuren hergestellt werden, falls die Austauschgruppen X basische Gruppen (zum Beispiel Aminogruppen) enthalten.

Falls X ein Wassermolekül bedeutet, kommen für die Neutralisierung der positiven Ladung des Platinatoms die genannten Säuren in Frage, insbesondere starke Säuren, vorzugsweise $H_2SO_4$.

Die Formeln I und II umfassen auch die möglichen Enantiomere und Diastereomere. Falls die Verbindungen Racemate sind, können diese in an sich bekannter Weise, zum Beispiel mittels einer optisch aktiven Säure oder mittels chiraler Phasen in die optisch aktiven Isomere gespalten werden. Es ist aber auch möglich, von vornherein enantiomerenreine oder gegebenenfalls auch diastereomerenreine Ausgangsstoffe einzusetzen, wobei dann als Endprodukt eine entsprechende reine optisch aktive beziehungsweise diastereomere Verbindung erhalten wird. Unabhängig von der Struktur der Reste X besitzt auch der Platinligand asymmetrische Kohlenstoffatome und kann daher in der Racemat-Form oder in optisch aktiven beziehungsweise diastereomeren Formen vorliegen.

In Bezug auf das Platinatom handelt es sich bei den erfindungsgemäßen Verbindungen der Formel I insbesondere um die cis-Verbindungen.

Das Ausgangsamin II wird beispielsweise als Racemat, als reine rechts- beziehungsweise linksdrehende Form als cis- oder trans-Form (in Bezug auf die Stellung der Aminomethylgruppen) oder in einer sonstigen diastereomeren Form eingesetzt.

Diese Konfigurationen bleiben bei der Herstellung des Platinkomplexes erhalten.

Das Verfahren zur Herstellung der erfindungsgemäßen Platin(II)-Komplexe der Formel I wird in einem Lösungsmittel bei Temperaturen zwischen 10 und 80°C, vorzugsweise 20 bis 40°C, insbesondere 25 bis 30°C durchgeführt. Als Lösungsmittel kommen beispielsweise in Frage: Wasser, $C_1$-$C_6$-Alkanole (Methanol, Ethanol, tert.-Butanol), cyclische Ether wie Tetrahydrofuran, Dioxan, gesättigte Ether von ein- oder mehrwertigen Alkoholen wie Ethylenglykoldimethylether, Diethylenglykoldimethylether, niedere gesättigte Ketone (Aceton, Methylethylketon), aprotische Mittel wie Dimethylsulfoxid oder Dialkylamide von niederen aliphatischen Carbonsäuren (Ameisensäure, Essigsäure) mit $C_1$-$C_6$-Alkylresten wie Dimethylformamid, Dimethylacetamid sowie Gemische dieser Lösungsmittel, insbesondere Mischungen mit Wasser.

Die beiden Reaktionskomponenten (Platin-Verbindung und Verbindung II) werden vorzugsweise in äquimolaren Mengen eingesetzt. Der pH-Wert der Reaktionslösung soll zwischen 5 und 9, vorzugsweise bei pH 6 liegen. Die Einstellung des pH-Wertes erfolgt insbesondere durch Zusatz von Alkali, vorzugsweise wäßriger Natronlauge oder Kalilauge oder beispielsweise auch mittels Natriumcarbonat beziehungsweise durch Zusatz von Säuren, vorzugsweise wäßriger Salzsäure. Die Einstellung des pH-Wertes kann auch mittels Ionenaustauschern erfolgen.

Als Tetrahalogen-platin(II)-Verbindungen (Säure sowie Komplexsalze) kommen die entsprechenden

Tetrachloro-, Tetrabromo- und Tetrajodo-Verbindungen in Frage. Falls Platin(II)-halogenide als Ausgangskomponente eingesetzt werden, kommen die gleichen Halogenatome in Frage.

Als einwertige Kationen kommen in Betracht: Alkali-Ionen, insbesondere Natrium und Kalium; es können aber auch Lithium, Rubidium, Cäsium verwendet werden, ebenso $NH_4^+$, $NR_4^+$, $PR_4^+$ oder $AsR_4^+$, in denen R ein $C_1$-$C_6$-Alkylrest oder ein Phenylrest ist. Zweiwertige Kationen können sein: Erdalkali-Ionen, insbesondere $Mg^{2+}$ und $Ca^{2+}$, aber auch $Zn^{2+}$. Als Platin(II)-halogenide kommen beispielsweise $PtCl_2$, $PtBr_2$ und $PtJ_2$ in Frage.

Die Verbindung II wird entweder in Form des Diamins oder in Form eines Säureadditionssalzes eingesetzt: zum Beispiel als Monohydrochlorid oder Dihydrochlorid, Mono- oder Dihydrobromid, Mono- oder Dihydrojodid oder als Salz mit einer anderen üblichen anorganischen oder organischen Säure. Insbesondere kommen auch die Säuren in Frage, deren Anionen die Reste X bilden. Weiterhin kann das Diamin in Form des Acetats beziehungsweise Diacetats eingesetzt werden, wobei gegebenenfalls vor dem Mischen der Reaktionskomponenten Kaliumchlorid (beispielsweise 2 Mol pro 1 Mol Verbindung II) zugesetzt wird. Ebenso kann das Diamin II beispielsweise in Form des Hydrochlorids, Carbonats, Oxalats oder Malonats eingesetzt werden.

Die Herstellung von Ethylendiamin Liganden der Formel II erfolgt beispielsweise durch Reduktion einer Verbindung der Formel

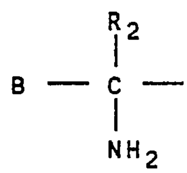

III

oder von deren Salzen, wobei Z entweder die Cyangruppe oder die Gruppe $-CONH_2$ ist oder die Gruppe

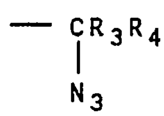

darstellt und B sowie die Reste $R_2$, $R_3$ und $R_4$ die angegebenen Bedeutungen haben.

Dieses Verfahren wird in einem Lösungs- oder Suspensionsmittel bei Temperaturen durchgeführt, die beispielsweise zwischen 20 bis 150°C, vorzugsweise 40 bis 150°C liegen. Gegebenenfalls kann auch unter erhöhtem Druck (bis zu 150 bar) gearbeitet werden. Als Lösungs- oder Suspensionsmittel kommen beispielsweise in Betracht: Wasser, symmetrische oder unsymmetrische Alkylether mit Alkylgruppen von 1 - 6 C-Atomen, gesättigte cycloaliphatische Ether wie Tetrahydrofuran, Dioxan, $C_1$-$C_6$-Alkanole (Methanol, Ethanol, Isopropanol), niedere Alkylamide beziehungsweise Dialkylamide von aliphatischen $C_1$-$C_2$-Carbonsäuren, cyclische aliphatische Säureamide (5- oder 6-Ring) wie N-Methyl-pyrrolidon sowie Mischungen dieser Mittel.

Als Reduktionsmittel kommen in Betracht: Katalytisch aktivierter Wasserstoff unter Verwendung üblicher Metallkatalysatoren (mit und ohne Träger), wie Edelmetallkatasysatoren (Palladium, Palladiumkohle, Palladium auf Bariumsulfat, Platin, Platinoxid, Rhodium, Ruthenium) oder auch Nickel und Cobalt-Katalysatoren, beispielsweise Gemische solcher Katalysatoren. Die Nichtedelmetallkatalysatoren können ebenfalls metallisch auf Trägern (zum Beispiel auf $SiO_2$, Kieselgur, $Al_2O_3$) oder insbesondere in aktivierter Form (zum Beispiel des Typs Raney) angewendet werden. Die Katalysatormenge kann zum Beispiel auch im Überfluß verwendet werden, zum Beispiel 1 - 80 %, vorzugsweise 2 - 40 %, insbesondere 10 - 30 % der eingesetzten Ausgangsverbindung. Als Lösungsmittel kommen hierbei vorzugsweise polare Mittel, wie Alkohole oder Alkohol-Wasser-Mischungen in Betracht. Die Reduktion ist aber auch möglich mit Leichtmetallhydriden, insbesondere komplexen Leichtmetallhydriden (Natriumhydrid, Lithiumhydrid, Lithiumaluminiumhydrid, Natriumtriäthoxy-aluminiumhydrid, Natrium-bis-2-methoxy-ethoxy-aluminiumdihydrid, Lithium-tri-tert.-butoxy-aluminiumhydrid und ähnlichen), wobei als Lösungsmittel vorzugsweise alicyclische oder cyclische Ether erwendet werden bei Temperaturen von vorzugsweise 20 - 100°C.

Falls die Reduktion mit Wasserstoff im Gegensatz von üblichen Hydrierungskatalysatoren erfolgt, kann auch unter Druck gearbeitet werden. Beispielsweise kommen Drucke von 1 - 300 bar, vorzugsweise 150 bar

insbesondere 70 - 100 bar in Frage.

Falls es sich bei den Ausgangsverbindungen der Formel III um Nitrile oder Carbonsäureamide (-$CONH_2$) handelt, erfolgt die Reduktion vorzugsweise mit einem komplexen Metallhydrid (zum Beispiel $LiAlH_4$) in einem cyclischen Ether (Tetrahydrofuran) bei Temperaturen zwischen 40 -100°C oder mittels Wasserstoff in Gegenwart eines Platin-Katalysators. Falls als Ausgangsverbindung III ein Azid reduziert wird, erfolgt diese Reduktion vorzugsweise ebenfalls mit einem komplexen Metallhydrid wie $LiAlH_4$ bei Temperaturen zwischen 10 und 50°C (Lösungsmittel insbesondere aliphatische gegebenenfalls wasserhaltige Ether wie zum Beispiel feuchter Diethylether).

Falls die Reaktion mit komplexen Metallhydriden erfolgt, ist anschließend eine Hydrolyse erforderlich. Diese erfolgt zum Beispiel mit Wasser oder wasserhaltigen cyclischen oder nicht-cyclischen Ethern (zum Beispiel Diethylether) bei Temperaturen zwischen 0 - 30°C, vorzugsweise 0 - 20°C insbesondere 0 - 5°C.

Ein weiterer Weg zur Herstellung von Diaminliganden der Formel II ist der folgende:

Ein Keton der allgemeinen Formel

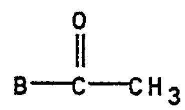

B, zum Beispiel Phenylalkyl-Rest (z.B. Phenylethyl)

wird in bekannter Weise mit Blausäure und Ammoniumcarbonat zu einem Hydantoin der allgemeinen Formel

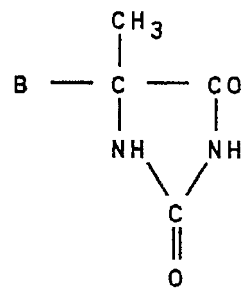

cyclisiert.

Temperaturbereiche:

| | |
|---|---|
| 0 - 50°C, | vorzugsweise 20°C für die Zugabe der Blausäure |
| 10 - 80°C, | vorzugsweise 60°C für die nachfolgende Reaktionszeit |

Ringöffnung

Die Ringöffnung im Autoklaven erfolgt vorzugsweise drucklos (Eigendruck entwickelt sich, wenige bar) bei Temperaturen von 100 - 250°C, vorzugsweise 180°C.

Veresterung

mit $SOCl_2$ wie bei der Herstellung der Ausgangssubstanzen beschrieben.

Darstellung des Amids

im Glas-Autoklaven (oder anderem Metallautoklav) alkoholischer (methanolisch am günstigsten) Lösung, die mit Ammoniak gesättigt ist.

Temperatur: -20°C bis +50°C (vorzugsweise Raumtemperatur)

Reduktion des Amids zum Diamin

mit $LiAlH_4$ (Zugabetemperatur beispielsweise -10°C) in Diethylether oder Tetrahydrofuran.

Fällung des Amins vorzugsweise als Oxalat oder Hydrochlorid.

Als Lösungsmittel kommen Alkohol/Wasser-Mischungen oder Methanol/Wasser in Frage.

Darstellung von 5-Methyl-5-phenethyl-hydantoin
= (5-Methyl-5-phenethyl-imidazolin-2,4-dion)

In einem 2-l-Dreihalskolben wird eine Suspension von 144,1 g (1,5 mol ) Ammoniumcarbonat in 800 ml Methanol/Wasser 1:1 Mischung hergestellt. Bei einer Temperatur von 20°C werden 43,2 ml (1,1 mol ) Blausäure innerhalb von 1 Minute zugegeben. Danach werden 152,8 g (1,0 mol) 4-Phenyl-2-butanol (Benzylaceton) zugetropft, was zu einer geringfügig exothermen Reaktion (Temperaturerhöhung auf ca. 30°C) führt. Die weiße Emulsion wird 1 Stunde bei 40°C gehalten, sodann 2,5 Stunden bei 60°C. Der Niederschlag wird abgesaugt, zweimal mit 50 ml Methanol/$H_2O$ 1:1 gewaschen und im Vakuum bei 60°C getrocknet.
Ausbeute: 212,3 g 97,3 % der Theorie
Schmelzpunkt: 178 - 179°C

α-Methyl-homo-phenylalanin

54,6 g 5-Methyl-5-phenethyl-hydantoin (0,25 mol) werden mit 30 g Natriumhydroxid (0,75 mol) und 150 ml Wasser 3,5 Stunden im Autoklaven bei einer Ölbadtemperatur von 180°C umgesetzt. Nach Abkühlung werden 350 ml $H_2O$ zugegeben und die klare, hellbraune Lösung mit Salzsäure auf pH 7,0 eingestellt. Der weiße Niederschlag wird abgesaugt, mit 20 ml $H_2O$ gewaschen und im Vakuumtrockenschrank bei 65°C getrocknet. Das Produkt wird zweimal aus Wasser umkristallisiert.
Ausbeute: 46,2 g 95,7 % der Theorie

α-Methyl-homo-phenylalanin-methylester

430 ml Mehanol werden auf -10°C in einer Eis/Kochsalzmischung abgekühlt und mit 21,3 ml Thionylchlorid versetzt (exotherme Reaktion). 44,5 g (0,23 ml ) α-Methyl-homo-phenylalanin werden bei Raumtemperatur zugetropft. Es wird 8,5 Stunden am Rückfluß gekocht, die Mischung am Rotationsverdampfer eingeengt, mit $H_2O$ aufgenommen (350 ml) und mit 150 ml 25 % $NH_4OH$-Lösung versetzt. Es wird 5 x mit Dichlormethan ausgeschüttelt und die vereinigten Extrakte über Kaliumcarbonat getrocknet und am Rotationsverdampfer eingeengt. Gelbliches Öl.
Ausbeute: 32 g 67 % der Theorie

α-Methyl-homo-phenylalanin-amid

32 g α-Methyl-homo-phenylalanin-methylester (0,15 mol) werden in einem Glasautoklaven 2 Tage mit 2 l gesättigter ammoniakalischer Methanol-Lösung bei Raumtermperatur umgesetzt. Es wird eingeengt und der Rückstand mit 300 ml Diethylether gewaschen.
Weiße Kristalle
Ausbeute: 18,6 g (62,6 % der Theorie)

1-Phenethyl-1-methylethylendiamin-Oxalat

240 ml trockenes Tetrahydrofuran werden in einer Eis/Kochsalz-Mischung auf -10°C abgekühlt. 10,96 g (0,289 mol) Lithiumaluminiumhydrid werden vorsichtig eingetragen. Bei -10°C werden vorsichtig 18,5 g Amid zugegeben, 30 Minuten in der Kälte rühren lassen und danach zum Sieden erhitzt. Nach 6,5 Stunden wird auf -10°C abgekühlt und 19 ml Essigsäureethylester, danach 41 ml Wasser vorsichtig zugetropft. Es wird abgesaugt, mit Tetrahydrofuran gewaschen und eingedampft. Das Amin wird in 100 ml absontem Alkohol aufgenommen und durch Versetzen mit 8,64 g Oxalsäure als Oxalat ausgefällt.
Ausbeute: 13,4 g

Das Verfahren zur Herstellung der Platin(IV)-Komplexe der Formel I erfolgt beispielsweise in den gleichen Mitteln wie bei dem Verfahren zur Herstellung der Platin(II)-Komplexe der Formel I. Diese Umsetzungen erfolgen hier in einem Temperaturbereich zwischen 20 und 100°C, vorzugsweise 40 - 80°C. Als Oxidationsmittel kommen in Frage: Halogene wie Chlorgas, Brom, Jod, Wasserstoffperoxid (zum Beispiel 3 bis 60%ig; vorzugsweise 10 bis 40 %, insbesondere 35 %), Dirhodan (gasförmig), Halogenwasserstoffsäuren (HCl, HBr, HJ). Falls die Oxidation mit Halogen, Dirhodan oder Halogenwasserstoffsäuren erfolgt, ist die zusätzliche Anwesenheit einer Verbindung HX gegebenenfalls nicht erforderlich.

Bei den Platin(IV)-Komplexen sind vorzugsweise 2 Reste x jeweils entweder Halogen oder Hydroxy. Im Falle der Platin(II)-Komplexe handelt es sich um planare quadratische Komplexe; im Falle der Platin(IV)-Komplexe steht dann von den 2 neuen zusätzlichen Substituenten x der eine Rest x oberhalb, der andere Rest x unterhalb der planaren Ebene.

Der Austausch der Liganden X gegen andere Liganden kann beispielsweise mittels der Silberhalogenidfällung erfolgen. Hierzu wird beispielsweise eine Dihalogenoplatin(II oder IV))-Verbindung der Formel I, worin X Halogen (Chlor, Brom oder Jod) bedeutet in einem Lösungs- oder Suspensionsmittel bei Temperaturen zwischen 0 bis 90°C, vorzugsweise 10 bis 50°C, insbesondere 30 bis 40°C, vorzugsweise 40°C mit den Silbersalzen einer anderen Säure, die der Bedeutung X entspricht, umgesetzt. Man kann hierbei aber auch als Silbersalz Silbernitrat (zum Beispiel wäßrige Silbernitrat-Lösung) verwenden und erhält, beispielsweise wenn es sich bei dem Platin-Komplex der Formel I um einen Platin(II)-Komplex handelt, einen ionischen Diaquakomplex der Formel

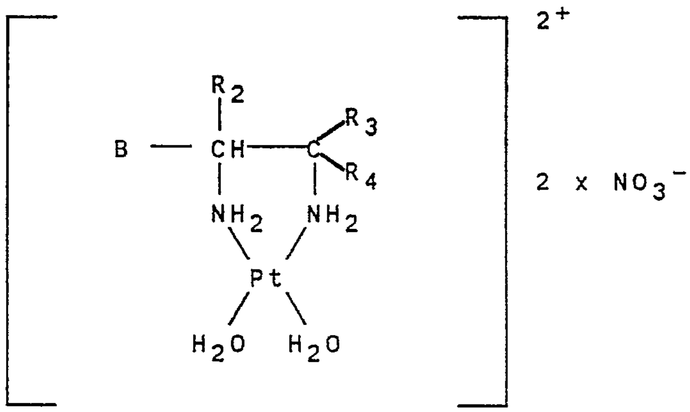

Aus diesem Komplex läßt sich der schwach gebundene Ligand Wasser leicht durch affinere Anionen (zum Beispiel $Cl^-$, $Br^-$ in Form von NaCl, KCl, NaBr, KBr, Malonat$^{2-}$, Chloracetat$^{(-)}$, Oxalat$^{2-}$, 1,1-Cyclobutan dicarbonsäure-Anion$^{2-}$, Glycolat, Lactat und Mandelat sowie die übrigen angegebenen Säurereste X verdrängen, angewandt in Form der Säuren oder ihrer Salze, insbesondere ihrer Alkalisalze.

Die gleichen Verbindungen lassen sich auch wie folgt gewinnen: Behandeln des zuvor angegebenen Diaqua-Nitrat-Komplexes mit einem Anionenaustauscher in der Hydroxidform (zum Beispiel Dowex 1 - 8X), wobei die 2 Moleküle Wasser durch OH ersetzt werden und anschließende Umsetzung der so erhaltenen Komplexverbindung (X = jeweils OH) mit der äquimolaren Menge HX wobei X ein physiologisch verträgliches Säureanion ist.

Ein Austausch der Abgangsgruppe (zum Beispiel $SO_4{}^{2-}$-beziehungsweise Oxalatanion$^{2-}$) ist im Falle der Sulfato- beziehungsweise Oxalato-platin(II)-Verbindungen auch durch Umsetzung mit Erdalkalisalzen, die den gewünschten X-Liganden (zum Beispiel Glycerinsäure) enthalten, möglich, sofern der entstehende Komplex wasserlöslich ist und damit die Abtrennung des schwer wasserlöslichen Erdalkalisulfats oder -oxalats erlaubt. Für dieses Verfahren geeignete X-Liganden sind vorzugsweise die Anionen von Hydroxycarbonsäuren, Sulfonsäuren, Halogenessigsäuren, Salpetersäure.

Die Lösungs- beziehungsweise Suspensionsmittel, die für das Herstellungsverfahren der Verbindungen I angegeben wurden, kommen auch für die Austauschreaktion in Frage (insbesondere eignen sich Wasser, Dimethylformamid, Dimethylacetamid, Dimethylsulfoxid, Methanol, Ethanol, tert.-Butanol, Aceton, Methylethylketon). Die Austauschreaktion wird beispielsweise in einem pH-Bereich zwischen 3 und 9 durchgeführt.

Die Wasserlöslichkeit beziehungsweise die Löslichkeit in physiologisch verträglichen Mitteln oder Mischungen hiervon untereinander und/oder mit Wasser (wässrige NaCl, Polyethylenglykole) der erfindungsgemäßen Platinkomplexe kann durch die Verwendung von lösungsvermittelnden Zusätzen verbessert werden. Solche Lösungsvermittler sind beispielsweise Cyclodextrine, Polyvinylpyrrolidon sowie nichtionogene Emulgatoren, die unter der Handelsbezeichnung Cremophor® bekannt sind (siehe H. P. Fiedler, Lexikon der Hilfsstoffe für Pharmazie, Kosmetik und angrenzende Gebiete von 1971, Verlag Cantor/Aulendorf in Württemberg, Seite 132-134).

Man erhält so echte Lösungen oder gegebenenfalls auch Suspensionen beziehungsweise Emulsionen.

Cyclodextrine sind cyclische Oligosaccharide, die durch enzymatischen Abbau der Stärke gewonnen werden. Man isoliert als Hauptabbauprodukte $\alpha$-, $\beta$-, und -Cyclodextrin, bei denen sechs ($\alpha$), sieben ($\beta$) oder acht ($\gamma$) D-($\alpha$)-Glucopyranose-Einheiten in ihrer Sesselform $\alpha$-1,4-glykosidisch verknüpft sind und die einen inneren Hohlraum aufweisen.

Dieser Hohlraum steht für die Bindung von unpolaren Substraten zur Verfügung, die durch den Einschluß in

den hydrophilen Mantel wasserlöslich werden. Die Höhle weist einen Durchmesser von 0,45 nm für α-Cyclodextrin, 0,7 nm für ß-Cyclodextrin und 0,8 -0,9 nm für γ-Cyclodextrin auf.
Im Fall der Platin-Komplexe muß ein Teil des Moleküls in den Hohlraum eintauchen, um eine Lösungsvermittlung zu gewährleisten. Der erfindungsgemäße Platin-Komplex wird für eine solche Präparation beispielsweise in Ethanol gelöst und mit der wäßrigen Lösung des Cyclodextrins vereinigt. Das Lösungsmittel wird dann wieder entfernt. Der verbleibende glasige Rückstand ist nach der Trocknung dann wasserlöslich.

Bei Platin-Komplexen, die in Ethanol oder anderen organischen Lösungsmitteln wenig löslich oder sogar unlöslich sind, wird beispielsweise die wäßrige α-Cyclodextrin-Lösung zur Suspension des Platin-Komplexes gegeben und die so erhaltene Mischung im Wasserbad auf 60°C erwärmt bis eine klare Lösung entsteht.

Die erreichbaren Löslichkeiten sind zum Beispiel in der folgenden Tabelle zusammengestellt.

Tabelle

Die angegebenen Mengen sind in 10 ml Lösungsmittelgemisch aus 1,8%iger NaCl-Lösung und Polyethylenglykol 400 (1:1) vollständig löslich.

Verwendung von α-Cyclodextrin in doppelt äquivalenter Menge zum Platin-Komplex

| Verbindung gemäß Beispiel | ohne Cyclodextrin | mit Cyclodextrin 2:1 |
|---|---|---|
| 2 | 6 mg | 18 mg |

Dieses Lösungsmittelgemisch wurde gewählt, weil es beispielsweise bei der Applikation in den in-vivo-Versuchen Verwendung findet.

Ein Erhöhen des PEG-Anteils im Lösungsmittelgemisch verbessert das Lösungsverhalten. Aus physiologischen Gründen ist dies jedoch für die zu applizierenden Präparationen nicht möglich.
Das Mengenverhältnis von Cyclodextrin und Platin-Komplex ist vorzugsweise 2:1.

Polyvinylpyrrolidon

Polyvinylpyrrolidon (PVP) ist ein wasserlösliches Polymeres, das in der pharmazeutischen Technologie unter anderem als viskositätserhöhende Substanz, als Gelbildner und Tablettierhilfsstoff Verwendung findet. Es kommen Polyvinylpyrrolidone mit Molgewichten von 5000 bis 400000 in Frage.

Mit den erfindungsgemäßen Platin-Komplexen entstehen Kopräzipitate. Zur Herstellung derselben wird der Platin-Komplex zunächst gelöst, und zwar in Ethanol oder einem anderen organischen Lösungsmittel. Die Lösung des Platin-Komplexes wird dann mit der ethanolischen PVP-Lösung versetzt und das Lösungsmittel abgezogen.
Die PVP-Ketten verhindern dabei die Ausbildung von Wechselwirkungen zwischen den Platin-Komplex-Molekülen, so daß ein gelbliches Glas entsteht, in dem der Komplex in der PVP-Matrix verteilt ist.
Der Platin-Komplex liegt hierbei unverändert in der PVP-Matrix vor. Das Anion X (Abgangsgruppe) wird bei der Kopräzipitation nicht ausgetauscht.

In der folgenden Tabelle sind zum Beispiel die Mengen an Platin-Komplex aufgeführt, die zum Beispiel als Kopräzipitat in 10 ml 1,8%iger Kochsalzlösung/Polyethylenglykol 400 (1:1) gelöst werden können.

Tabelle

Die angegebenen Mengen sind in 10 ml Lösungsmittelgemisch aus 1,8%iger NaCl-Lösung und Polyethylenglykol 400 (1:1) vollständig löslich.

Verwendung von PVP im Verhältnis 50:1 gegenüber dem Platin-Komplex

| Verbindung gemäß Beispiel | ohne PVP | als Kopräzipitat |
|---|---|---|
| 10 | 8,5 mg | 18 mg |
| 11 | 4,5 mg | 18 mg |
| 12 | keine Lösung | <5 mg |
| 2 | 10 mg | <18 mg |

Das Verhältnis von PVP zum Platin-Komplex wird für das Präzipitat so gewählt, daß 50 monomere Einheiten von PVP auf 1 Platin-Komplex-Molekül kommen. Das gewählte PVP wies ein durchschnittliches Molekulargewicht von 10 000 auf.

Die Herstellung von Lösungen, Suspensionen oder Emulsionen der erfindungsgemäßen Platin(II)-Komplexe unter Verwendung der Lösungsvermittler erfolgt beispielsweise durch Erwärmen der Komponenten auf Temperaturen zwischen 40 und 100°C, vorzugsweise 50 - 80 °C, gegebenenfalls unter Rühren oder Schütteln oder mittels Ultraschall in Wasser oder einem üblichen organischen Lösungsmittel (insbesondere Ethanol) oder Mischungen hiervon. Das Lösungsmittel kann dann durch Abziehung unter Vakuum oder Verdampfen entfernt werden. Dies ist zum Beispiel erforderlich, wenn das Lösungsmittel/Lösungsmittelgemisch nicht physiologisch verträglich ist. Der erhaltene Rückstand wird dann gegebenenfalls unter Rühren, Schütteln oder mittels Ultraschall in einem physiologisch verträglichen Lösungsmittel oder Suspensionsmittel gegebenenfalls unter Erwärmen auf 40 - 100 °C, vorzugsweise 40 - 80 °C wieder aufgenommen.

Die erfindungsgemäßen Verbindungen besitzen tumorhemmende Eigenschaften und sind zur Chemotherapie von Tumorerkrankungen geeignet. Beispielsweise wird mit den erfindungsgemäßen Verbindungen bei der lymphatischen Leukämie P 388 der weiblichen Maus eine mediane Überlebenszeit ausgedrückt in Prozent (% T/C, Definition siehe unten) von über 125 %, beispielsweise von 200 - 300 % erzielt. Komplexe der erfindungsgemäßen Verbindungen mit Hämatoporphyrin zeigen beispielsweise an der P 388/Leukämie eine hohe Antitumorwirkung bei $1 \times 10^{-5}$ bis $4 \times 10^{-5}$ Mol/kg Körpergewicht Maus.

Die Untersuchung auf Antitumorwirkung an der P 388-Leukämie der Maus erfolgt gemäß folgender Methode: Die lymphatische Leukämie P 388 wurde 1955 am National Cancer Institut der USA etabliert. Die Tumorlinie wurde durch lokales Auftragen von Methylcholanthren in DBA/2-Mäusen induziert und in der ersten Passage in die Ascites-Form überführt. (Dawe, Potter, Am. Ass. Scientific Proceedings; Patologists and Bacteriologists, 33 (1957), 603).

Die Stammerhaltung erfolgt auf weiblichen DBA/2-Mäusen. Da die mittlere Überlebenszeit dieses Tumors nur etwa zehn Tage beträgt, muß der Tumorstamm wöchentlich transplantiert werden. Dazu wird das tumortragende Tier durch Genickbruch getötet und unter sterilen Bedingungen der milchig trübe Ascites durch Punktion an der Bauchseite entnommen. Die Tumorzellen werden mit sterilfiltrierter, eiskalter PBS-Lösung (phosphatebuffered saline) auf eine Zellzahl von $1 \times 10^6$ /0,1 ml Injektionsvolumen verdünnt und 6 - 10 Wochen alten Tieren intraperitoneal implantiert.

Die Testung der Substanzen erfolgt auf weiblichen $CD_2F_1$-Mäusen (Zentralinstitut für Versuchstiere, Hannover) mit einem Körpergewicht von 17 - 21 g und einem Alter von 6 - 10 Wochen. Der Tumor wird wie für die Stammerhaltung beschrieben, am Tag 0 des Testes entnommen und transplantiert. Anschließend werden die Tiere in Gruppen zu je sechs Mäusen randomisiert.

Am folgenden Tag (Tag 1) sowie an Tag 5 und Tag 9, abgekürzt mit $d_1$, $d_5$, $d_9$, werden die zu testenden Substanzen in drei verschiedenen Konzentrationen ($1x10^{-5}$ Mol/kg; $2x10^{-5}$ Mol/kg und $4x10^{-5}$/ Mol/kg je einer Gruppe (pro Substanz also drei Gruppen nach Körpergewicht appliziert.
Pro Gramm Körpergewicht der Maus werden 0,01 ml einer Lösung gespritzt, die in 10 ml jeweils $1x10^{-5}$, $2x10^{-5}$ beziehungsweise $4x10^{-5}$ Mol Platin-Komplex, also die Menge für 1 kg Maus enthält.

Als Parameter für die Antitumorwirkung einer Substanz wird die sogenannte mediane Überlebenszeit der Testgruppe im Vergleich zur unbehandelten Kontrolle angegeben:

$$\frac{\text{mediane Überlebenszeit (Test)}}{\text{mediane Überlebenszeit (Kontrolle)}} \times 100 = \%\ T/C$$

Für die mediane Überlebenszeit gilt:
- bei gerader Tierzahl N:

$$\frac{x + y,}{2}$$

wobei x der Tag, an dem die Zahl der toten Tiere > N/2 und y der früheste Tag, an dem die Zahl der toten Tiere >(N/2)+1 ist.
- bei ungerader Tierzahl N: x

Nach dem NCI-Protokoll gilt eine Verbindung als antitumoraktiv, wenn der T/C-Wert größer als 125% ist. Weitere Aussagen ergeben sich aus dem Vergleich der Tiergewichte am Tag 1 und am Tag 5. Die Kontrolltiere nehmen in Folge des wachsenden Ascites 1,5 - 3 g zu. Umgekehrt weist eine Gewichtsabnahme auf toxische Nebenwirkungen hin. Eine Substanz gilt in der jeweiligen Konzentration als toxisch, wenn die Gewichtsabnahme größer als 4 g oder der T/C-Wert kleiner ist als 85%.

Die erfindungsgemäßen Verbindungen zeigen beispielsweise an der P388-Leukämie der Maus (Tumorimplantation intraperitoneal, Behandlung 1 x intraperitoneal) eine gute Antitumorwirkung. Beispielsweise wird bei obengenannter Versuchsmethode bei einer Dosis von 21,5 mg/kg Körpergewicht Maus eine Überlebenszeitverlängerung von 57 % erzielt.

Die niedrigste, bereits wirksame Dosis in dem obenangegebenen Tierversuch ist beispielsweise

2 mg/kg oral
0,5 mg/kg sublingual
0,5 mg/kg intravenös

Als allgemeiner Dosisbereich für die Wirkung (Tierversuch wie oben) kommt beispielsweise in Frage:

2 - 2000 mg/kg oral, insbesondere 50 - 500 mg/kg
0,5 - 1000 mg/kg intraperitoneal, insbesondere 3 - 100 mg/kg
0,5 - 1000 mg/kg intravenös, insbesondere 3 - 100 mg/kg

Die Wirkungsrichtung der erfindungsgemäßen Verbindungen ist mit der Wirkung des bekannten Arzneimittelwirkstoffs Cisplatin, Carboplatin vergleichbar, jedoch bestehen hierzu insbesondere folgende Unterschiede:
gegenüber Cisplatin: nahezu keine Nephrotoxizität
gegenüber Carboplatin: wesentlich geringere

Myelotoxizität und Hämatotoxizität

Indikationen für die die erfindungsgemäßen Verbindungen in Betracht kommen können:
Chemotherapie bösartiger Erkrankungen (Krebs)
Die pharmazeutischen Zubereitungen enthalten im allgemeinen zwischen 1 - 2000, vorzugsweise 10 - 1000

mg der erfindungsgemäßen aktiven Komponente(n).

Die Verabreichung kann beispielsweise in Form von Tabletten, Kapseln, Pillen, Dragees, Zäpfchen, Salben, Gelees, Cremes, Puder, Stäubepulver, Aerosolen, Lyophilisate, Pulver oder in flüssiger Form erfolgen. Als flüssige Anwendungsformen kommen zum Beispiel in Frage: Ölige oder alkoholische, beziehungsweise wässrige Lösungen, sowie Suspensionen und Emulsionen. Bevorzugte Anwendungsformen sind Tabletten, die zwischen 1 und 1000 mg, Lösungen die zwischen 5 und 200 mg oder Lyophilisate, die zwischen 5 und 400 mg, vorzugsweise 5 bis 200 mg an aktiver Substanz enthalten.

Die Einzeldosis der erfindungsgemäßen aktiven Komponenten kann beispielsweise liegen

a) bei oralen Arzneiformen zwischen 2 und 2000 mg, vorzugsweise 10 - 1000 mg.

b) bei parenteralen Arzneiformen (zum Beispiel intravenös, intramuskulär) zwischen 1 und 1000 mg, vorzugsweise 5 - 400 mg, insbesondere 5 bis 200 mg.

c) bei Arzneiformen zur rektalen oder vaginalen Applikation zwischen 2 und 2000 mg, vorzugsweise 10 - 1000 mg.

d) bei Arzneiformen zur lokalen Applikation auf die Haut und Schleimhäute (zum Beispiel in Form von Lösungen, Lotionen, Emulsionen, Salben und so weiter) zwischen 0,01 und 10 %, vorzugsweise 0,1 bis 2 %.

Beispielsweise können 3 mal täglich 1 - 4 Tabletten mit einem Gehalt von 10 bis 500 mg wirksamer Substanz oder zum Beispiel bei intravenöser Injektion 1 bis 4 mal täglich eine Ampulle bzw. Injektionsflasche von 0,5 bis 500 ml Inhalt mit 1 - 200 mg Substanz empfohlen werden. Bei oraler Verabreichung ist die minimale tägliche Dosis beispielsweise 1 mg; die maximale tägliche Dosis bei oraler Verabreichung soll nicht über 2000 mg liegen.

Für die Behandlung von Hunden und Katzen liegt die orale Einzeldosis im allgemeinen zwischen ungefähr 10 und 2000 mg/kg; die parenterale Einzeldosis ungefähr zwischen 1 und 1000 mg/kg Körpergewicht.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei intraperitonealer Applikation zwischen 20 und 2000 mg/kg (je nach Wirksubstanz).

Die Arzneimittel können in der Humanmedizin, der Veterinärmedizin, sowie in der Landwirtschaft allein oder im Gemisch mit anderen pharmakologisch aktiven Stoffen verwendet werden.

-(Die Dosen sind jeweils bezogen auf die freie Base)-

Herstellung der Ausgangssubstanzen für die Phenylalkylethylendiamin-Liganden

Ausgangssubstanzen für C2-substituierte Phenylalkylethylendiamine

Man geht zum Beispiel von einer Aminosäure der Formel

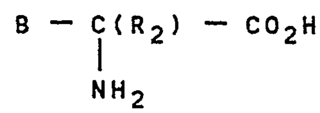

aus (zum Beispiel Phenylalanin beziehungsweise dem im Phenylkern entsprechend substituierten Phenylalanin) und verestert diese Verbindungen zunächst in an sich bekannter Weise mit Thionylchlorid und Methanol.

Durch Grignard-Reaktion werden die späteren Substituenten am 2-ständigen C-Atom des Benzylethylendiamins eingeführt und man erhält die entsprechenden Aminoalkohole der Formel

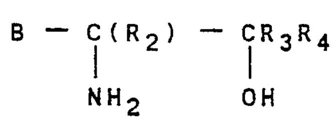

Der so erhaltene Aminoalkohol kann nun mit $Ph_3PBr_2$ und $Et_3N$ in Acetonitril durch eine trans-Eliminierung in das Aziridin überführt werden. Dazu lagert sich das Reagens an die OH-Gruppe des Alkohols an, und $Ph_3PO$ tritt schließlich unter Ringschluß aus.

$Ph_3PBr_2$ wird für die Reaktion in situ aus $Ph_3P$ und $Br_2$ hergestellt.

Die Öffnung des Aziridin-Ringes erfolgt mit $NaN_3$ unter Bildung des Aminoazides, welches dann mit $LiAlH_4$ zum Diamin-Liganden reduziert werden kann. Dieser Weg kommt insbesondere in Betracht falls die Reste $R_3$ und $R_4$ Alkylreste sind.

Falls die Reste $R_3$ und $R_4$ beispielsweise Phenyl, p-Anisyl oder Cyclohexyl sind, das heißt nichtlineare Reste, empfiehlt sich eine direkte Substitution der OH-Gruppe durch die Azidgruppe mit dem Dreikomponentensystem Azodicarbonsäurediethylester Triphenylphosphin und Alkohol unter Zusatz eines vierten Partners HX. Aus dem Triphenylphosphin und der Azo-Verbindung entsteht eine Betain-Struktur, die die OH-Gruppe der Alkoholkomponente stark für den Austritt aktiviert, so daß die Substitution mit dem Azid-Nukleophil aus der Stickstoffwasserstoff-Säure erfolgen kann. Die benötigte Stickstoffwasserstoff-Säure wird in Form einer Toluol-Lösung eingesetzt, deren Gehalt jeweils bestimmt werden muß.

Das Aminoazid wird schließlich isoliert, indem das Lösungsmittel zunächst vorsichtig abgezogen und der Rückstand auf eine Kieselgel-Säule gebracht wird. Die Chromatographie mit Ether-Toluol 1:1 trennt das Reaktionsprodukt in ausreichender Reinheit ab. Die so erhaltenen Ausgangsaminoazide haben die allgemeine Formel

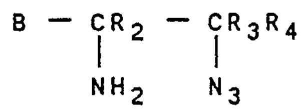

Um zum Diamin zu gelangen, wird wiederum mit $LiAlH_4$ reduziert. Im folgenden werden diese Reaktionen beispielhaft erläutert:

D,L-Phenylalaninmethylester-hydrochlorid

Ca. 150 ml Methanol werden vorgelegt und 115 mMol (13.68 g; 8.3 ml) $SOCl_2$ unter Eiskühlung aus einem Tropfrichter langsam zugetropft. Die 110 mMol (18.17 g) D,L-Phenylalanin werden als Feststoff zugegeben. Der Ansatz wird 24 Stunden am Rückfluß erhitzt. Nach dem Abziehen des Lösungsmittels erhält man einen farblosen Rückstand, der am Hochvakuum ($10^{-4}$ Torr) getrocknet wird. Das Esterhydrochlorid wird ohne weitere Reinigung weiterverarbeitet. Ausbeute: quantitativ.

Analog erhält man zum Beispiel

4-Chlorphenylalaninmethylester-hydrochlorid

4-Methoxyphenylalaninmethylester-hydrochlorid

Grignard-Reaktion

0,1 Mol D,L-2-Methyl-3-amino-4-phenylbutan-2-ol

(2.43 g) Mg-Späne werden mit Ether überschichtet. Aus dem Tropftrichter wird dann ca. 1/3 der 2M Lösung von 0.1 Mol (14.2 g; 6.2 ml) $CH_3I$ in Ether zugegeben.

Durch leichtes Erwärmen kann die Grignard-Reaktion zum Anspringen gebracht werden. Unter leichtem Rühren wird die restliche Methyljodid-Lösung so zugetropft, daß die Mischung gerade am Sieden gehalten wird. Nach vollständiger Zugabe wird noch ca. 30 Minuten zum Sieden erhitzt.

Nach Abkühlen werden im Eisbad 0.017 Mol (3.6 g) D,L-Phenylalaninmethylester-hydrochlorid als Feststoff portionsweise zugegeben. Die Mischung wird dann 15 Stunden zum Sieden erhitzt.

Die Hydrolyse erfolgt mit einer gesättigten Lösung von 0,17 Mol (9,1 g) $NH_4Cl$ in Wasser. Die Lösung wird vorsichtig unter kräftigem Rühren filtriert, um vom unlöslichen Hydrolyseprodukt zu befreien, und das Filtrat in einen Scheidetrichter überführt.

Die organische Phase wird über $Na_2SO_4$ getrocknet und dann das Lösungsmittel am Rotationsverdampfer abgezogen. Man erhält ein farbloses Öl.

Die wäßrige Phase wird mit konzentriertem $NH_3$ ammoniakalisch gemacht und mit Ether ausgeschüttelt. Nach dem Abziehen des Lösungsmittels erhält man ein öliges Produkt. Die öligen Rückstände aus organischer und wäßriger Phase werden im Hochvakuum ($10^{-4}$ Torr) bei 120° C kugelrohrdestilliert. Ausbeute: 70 % farbloses Öl.

Analog erfolgt die Grignard-Reaktion unter Verwendung anderer Alkylhalogenide, Cycloalkylhalogenide beziehungsweise aromatischen Halogenverbindungen (zum Beispeil Brombenzol, Bromanisol und so weiter).

Der Aziridin-Weg (D,L-2-Benzyl-3,3-dimethylaziridin)

40m Mol (10,48 g) $Ph_3P$ werden in 100 ml Acetonitril gelöst. Zu dieser Lösung werden langsam 40 mMol (6.40 g; 2.06 ml) $Br_2$ in 40 ml Acetonitril getropft. 40 mMol (7.17 g) des Aminoalkohols D,L-2-Methyl-3-amino-4-phenylbutan-2-ol werden in ca. 100 ml Acetonitril gelöst und zu der $Ph_3PBr_2$-Lösung getropft. Die Zugabe erfolgt unter Eiskühlung.
In diese Mischung werden langsam unter Rühren 80 mMol (8.08 g; 11.1 ml) $Et_3N$ in 10 ml Acetonitril getropft. Nach kurzer Zeit ist ein farbloser Niederschlag von $[Et_3NH]^+[Br]^-$ zu beobachten.

Nachdem der Ansatz über Nacht gestanden hat, filtriert man das Triethylamin-hydrobromid ab und engt das Filtrat am Rotationsverdampfer ein.
Der Rückstand wird in eine Flüssig/Flüssig-Extraktionsapparatur überführt und 24 Stunden mit 250 ml Hexan extrahiert. Der erneut gebildete Niederschlag von Triethylamin-hydrobromid wird abfiltriert und mit Hexan gewaschen. Vom Filtrat wird das Lösungsmittel am Rotationsverdampfer abgezogen und der Rückstand im Hochvakuum ($10^{-4}$ Torr) bei 90° C kugelrohrdestilliert.
Ausbeute: 40 % farbloses Öl.

Öffnung der Aziridine zu den Amino-Aziden (D,L-1-Phenyl-2-amino-3-azido-3-methylbutan)

10 mMol (1.61 g) des Aziridins werden in 40 ml Ethanol gelöst, mit 40 mMol (2.60 g) $NaN_3$ und 40 mMol (2.14 g) $NH_4Cl$ in 15 ml Wasser versetzt und 14-18 Stunden unter Rückfluß erhitzt. Dann wird mit Wasser verdünnt und mit $CH_2Cl_2$ ausgeschüttelt. Die organische Phase wird über $Na_2SO_4$ getrocknet und das Lösungsmittel schließlich abgezogen. Ausbeute 60 % dunkelgelbes Öl.

Der Azodicarbonsäurediethylester-Weg

Darstellung der Stickstoffwasserstoff-Säure:
0.1 Mol (6.5 g) $NaN_3$ werden in 6.5 ml destilliertem Wasser aufgenommen. Zu dieser Lösung werden 40 ml Toluol gegeben. Nach Abkühlung des Ansatzes auf 0° C werden im Eisbad 5.16 ml konzentrierte $H_2SO_4$ (95 %ig) so zugetropft, daß die Temperatur im Reaktionsgefäß +5° C nicht übersteigt.
Nach vollständiger Zugabe läßt man auf Raumtemperatur kommen und überführt in einen Scheidetrichter, um die Wasserphase abzutrennen. Die Toluolphase wird über $Na_2SO_4$ getrocknet. Der Gehalt der Toluol-Lösung an $HN_3$ wird mit 0.1N NaOH titrimetrisch bestimmt.

D,L-1,1-Dicyclohexyl-1-azido-2-amino-3-phenylpronan

Zu einer Lösung von 22 mMol (5.77 g) $Ph_3P$ und 20 mMol (6.4 g) D,L 1,1-Dicyclohexyl-2-amino-3-phenylpropan1-ol in 100 ml Toluol werden aus dem Tropftrichter 22 mMol (3.89 g) Azodicarbonsäurediethylester, gelöst in 20 ml Toluol, getropft.
Nach Zugabe von 22 mMol $HN_3$ in Toluol-Lösung entsteht ein farbloser Niederschlag. Der Ansatz wird 15 Stunden bei Raumtemperatur gerührt, schließlich der Niederschlag abfiltriert und das Lösungsmittel abgezogen.

Das bräunliche Öl, welches man als Rückstand erhält, wird erneut in wenig Toluol gelöst und auf eine Kieselgelsäule (50 cm x 5 cm) aufgetragen. Nach dem Einlaufen der Substanz in die Säule wird mit Toluol/Ether (1:1) chromatographiert. Man fängt das Eluat in vier Portionen zu ca. 70 ml auf.
Nach Abziehen des Lösungsmittels zeigen die IR-Spektren der Fraktionen 3 und 4 die Azid-Bande der Amino-Azid-Verbindung. Ausbeute: 30 % braun-gelbes Öl.

Die Herstellung anderer 1-Azido-2-amino-3-phenylpropane mit 2 Substituenten (zum Beispiel 2 gegebenenfalls substituierten Phenylresten) in 1-Stellung erfolgt analog.

Ausgangssubstanzen für C1-substituierte Phenylalkylethylendiamine.

Ausgehend von den entsprechenden Phenylalkyl-Ketonen können in hierfür üblicher Weise in einer Strecker-Synthese mit NaCN und $NH_4Cl/NH_3$ die Amino-Nitrile dargestellt und isoliert werden.

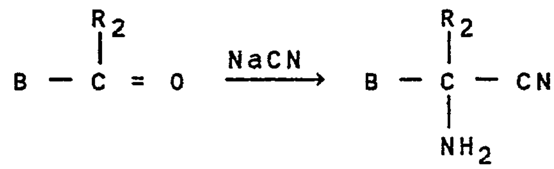


Die Isolierung erfolgt, indem nach Abschluß der Reaktion mit 1N HCl auf pH 6 gebracht und so das Amino-Nitril in sein Hydrochlorid überführt wird. Das noch vorhandene Keton kann dann mit Ether extrahiert werden, bevor mit Ammoniak das Amin freigesetzt wird, welches jetzt ebenfalls mit Ether extrahiert werden kann.
Die Reduktion des Amino-Nitrils mit $LiAlH_4$ liefert nach Hydrolyse dann die entsprechenden Diamine. Diese Reduktion führt in allen Fällen auch zu einem Nebenprodukt. Das gewünschte Diamin läßt sich jedoch in allen Fällen durch eine einfache Kugelrohrdestillation analysenrein gewinnen.

Allgemeine Vorschrift zur Herstellung der cis-Diamin dichloro-Platin(II)-Komplexe der Formel I

A Ausführung in Wasser

5 mMol des entsprechenden Diamins (Verbindung II) werden in ca. 25 ml destilliertem Wasser gelöst. Die Lösung wird mit 1N HCl auf pH 6 eingestellt. 5 mMol $K_2PtCl_4$ (2,08g) werden in 25 ml $H_2O$ gelöst und unter Rühren zur neutralen Diamin-Lösung gegeben. Der Ansatz wird im Wasserbad auf ca. 20° C erwärmt und der pH-Wert laufend kontrolliert. Falls notwendig wird mit 1N NaOH auf pH 6 eingestellt. Die Reaktion ist abgeschlossen, wenn sich der pH-Wert mit der Zeit kaum mehr ändert. Der Komplex fällt als feinkristalliner Niederschlag aus und wird abfiltriert (zum Beispiel über eine Membranfilterfritte). Dann wird mit Wasser und wenig Ethanol gewaschen und schließlich unter Hochvakuum ($10^{-4}$ Torr) getrocknet. Das Reaktionsprodukt kann zum Beispiel umkristallisiert werden, indem man 50 mg davon in 10 ml heißem Acetonitril löst, dann auf Raumtemperatur und schließlich in der Kühltruhe auf -10° C abkühlt.

B Ausführung in Wasser/tertiärem Butanol

0,5 mMol des entsprechenden Diamins werden in 50 ml tertiärem Butanol gelöst. Hierzu wird eine Lösung von 0,5 mMol $K_2PtCl_4$ in 5 ml Wasser gegeben. Es entsteht ein Niederschlag, der sich nach Zusatz von weiteren 25 ml Wasser auflöst. Die so entstandene Lösung wird mit 1N HCl neutralisiert. Nach einiger Zeit (zum-Beispiel nach 30 Minuten) scheidet sich das Reaktionsprodukt (Platin-Komplex) ab. Nach einigen Stunden (zum Beispiel 3 Stunden) wird der Niederschlag abgesaugt und getrocknet und gegebenenfalls, wie unter A angegeben, umkristallisiert.

Beispiel 1

D,L-1-(4-Chlorbenzyl)-2,2-dimethyl-ethylendiamindichloro-platin(II)
Ausführung A
Ausbeute: 76%, blaßgelbes Pulver; F. >300° C (Zersetzung)
$C_{11}H_{17}Cl_3N_2Pt$ (Molgewicht 478,7 g).
IR (KBr): 3240, 3110 $cm^{-1}$ (NH), 3040 (CH arom.), 2980 (CH aliph.), 1580 (NH), 330, 320 (PtCl).-
$^1$H-NMR ([$D_7$]DMF, 250 MHz): $\delta$ = 7.34-7.44 (AA'BB', 4H, phenyl), 5.43 (m, 4H, $NH_2$), 3.13 (m, 1H, CH-N), 2.73-3.00 (m, von Lösungsmittelsignalen verdeckt, $CH_2$-phenyl), 1.49 (s, 3H, $CH_3$), 1.40 (s, 3H, $CH_3$).
(Die Buchstaben AA'BB', AB bedeuten Kopplungsmuster für verschiedene Protonengruppierungen)

| Analysenwerte: | C | H | N |
|---|---|---|---|
| berechnet: | 27.60 | 3.58 | 5.85 |
| gefunden: | 27.58 | 3.50 | 5.76 |

Beispiel 2

D,L-1-Benzyl-2,2-dimethyl-ethylendiamindichloro-platin(II)
Ausführung A
Ausbeute: 37%, blaßgelbe Nadeln; F. >300° C (Zersetzung)
$C_{11}H_{18}Cl_2N_2Pt$ (Molgewicht 444.3 g).
IR (KBr): 3190, 3110 $cm^{-1}$ (NH), 3020 (CH arom.), 2980 (CH aliph.), 1570 (NH), 300 (PtCl). - $^1H$-NMR ($CD_3CN$, 250 MHz): $\delta$ = 7.21-7.29 (m, 5H, phenyl), 4.72, 4.44, 4.26, 4.07, 3.91 (5m, 4H, $NH_2$), 3.21 (m, 1H, $^3J_{CH-CH}$ = 3.8, $^3J_{CH-CH}$ = 11.0, $^3J_{CH-NH}$ = 3.8, $^3J_{H-NH}$ = 18.0, CH-N), 2.88 (AB, 1H, $^2J$ = 14.3, CH-phenyl), 2.61 (AB, 1H, CH-phenyl), 1.40 (s, 3H, $CH_3$), 1.31 (s, 3H, $CH_3$).

| Analysenwerte: | C | H | N |
|---|---|---|---|
| berechnet: | 29.74 | 4.08 | 6.31 |
| gefunden: | 29.82 | 4.18 | 6.23 |

Beispiel 3

D,L-1-Benzyl-2,2-dimethylethylendiamindichloro-platin(II)
Ausführung A
Ausbeute: 68%, blaßgelbes Pulver; F. >300° C (Zersetzung)
$C_{13}H_{22}Cl_2N_2Pt$ (Molgewicht 472.3 g).
IR (KBr): 3190, 3120 $cm^{-1}$ (NH), 3030 (CH arom.), 2970 (CH aliph.), 1590 (NH), 330, 320 (PtCl). - $^1H$-NMR ([$D_7$]DMF, 250 MHz): $\delta$ = 7.25-7.49 (m, 5H, phenyl), 5.37, 5.40 (2m, 1H, $NH_2$), 5.20, 5.25 (2m, 1H, $NH_2$), 5.06, 5.11 (2m, 1H, $NH_2$), 4.28, 4.32 (2m, 1H, $NH_2$), 2.93-3.12 (m, 3H, $CH_2$-phenyl, CH-N), 1.95-2.23, 1.72-1.91 (2m, 4H, $CH_2$), 0.97-1.08 (2t, 6H, $^3J$ = 7.6, $CH_3$).

| Analysenwerte: | C | H | N |
|---|---|---|---|
| berechnet: | 33.06 | 4.70 | 5.93 |
| gefunden: | 33.22 | 4.67 | 5.93 |

Beispiel 4

D,L-1-Benzyl-2,2-dipentyl-ethylendiamindichloro-platin(II)
Ausführung B
Ausbeute: 37%, blaßgelbes Pulver; F. >300° c (Zersetzung)
$C_{19}H_{34}Cl_2N_2Pt$ (Molgewicht 556.5 g).
IR (KBr): 3240, 3110 $cm^{-1}$ (NH), 3040 (CH arom.), 2960, 2940 (CH aliph), 1580 (NH), 330, 320 (PtCl ). - $^1H$-NMR ([$D_7$]DMF, 250 MHz): $\delta$ = 7.17-7.34 (m, 5H, phenyl), 5.54, 5.58 (2m, 1H, $NH_2$), 4.97, 3.69 (2m, 2H, $NH_2$), 3.48, 3.52 (2m, 1H, $NH_2$), 2.93-2.99 (m, 2H, $^2J$ = 11.1, $^3J$ = 14.8, CH-phenyl, CH-N), 2.45 (AB, 1H, CH-phenyl), 1.25-1.69 (m, 16H, $CH_2$), 0,95 (t, 3H, $CH_3$), 0,88 (t, 3H, $CH_3$).

| Analysenwerte: | C | H | N |
|---|---|---|---|
| berechnet: | 41.01 | 6.16 | 5.04 |
| gefunden: | 41.43 | 5.93 | 4.66 |

Beispiel 5

D,L-1-Benzyl-2,2-dicyclohexyl-ethylendiamindichloro-platin(II)
Ausführung B
Ausbeute: 32%, blaßgelbes Pulver; F. >300° C (Zersetzung)

$C_{21}H_{34}Cl_2N_2Pt$ (Molgewicht 580.5 g). Der Komplex kann aus der Acetonitril-Lösung durch Etherzusatz bei Raumtemperatur ausgefällt werden.
IR (KBr): 3200 $cm^{-1}$ (NH), 3040 (CH arom.), 2940, 2860 (CH aliph.), 1610 (NH), 320 (PtCl).

| Analysenwerte: | C | H | N |
|---|---|---|---|
| berechnet: | 43.45 | 5.90 | 4.83 |
| gefunden: | 43.15 | 4.60 | 5.13 |

Beispiel 6

D,L-1-Benzyl-2,2-diphenyl-ethylendiamindichloro-platin(II)

5 mMol (1.51 g) D,L-1-Benzyl-2,2-diphenyl-ethylendiamin werden mit soviel 1N HCl versetzt, daß das halbfeste Diamin gelöst wird. Diese Lösung wird mit 1N NaOH neutralisiert, wobei eine trübe Emulsion entsteht.

5 mMol (2.08 g) $K_2PtCl_4$, in 25 ml $H_20$ gelöst, werden zu dieser Emulsion gegeben. Bei einem konstant gehaltenen pH-Wert von 6.5 - 7 wird ca. 5 Stunden gerührt, bis der ausgefallene Komplex abfiltriert werden kann.
Der Niederschlag wird mit Ether und destilliertem $H_2O$ gewaschen und am Hochvakuum ($10^{-4}$ Torr) getrocknet.
Ausbeute: 56%, blaßgelbes Pulver F. >300° C $C_{21}H_{22}Cl_2N_2Pt$ (Molgewicht 568.4 g).
IR (KBr): 3220, 3160 $cm^{-1}$ (NH) 3110 (CH arom.), 2960 (CH aliph.), 1580 (NH), 330, 320 (PtCl). -
$^1$H-NMR ([$D_7$]DMF, 250MHz): $\delta$ = 7.95-8.13 (m, 5H, phenyl), 7.33-7.56 (m, 10H, phenyl), 6.62 (m, 2H, $NH_2$), 4.94, 5.00 (2m, 1H, $NH_2$), 4.32, 4.38 (2m, 1H, $NH_2$), 3.92 (m, 1H, $^3J$<3, $^3J$ = 14.8, CH-N), 3.26 (AB, 1H, $^2J$ = 11.1, CH-phenyl), 3.11 (AB, 1H, CH-phenyl).

| Analysenwerte: | C | H | N |
|---|---|---|---|
| berechnet: | 44.36 | 3.90 | 4.93 |
| gefunden: | 44.13 | 4.11 | 4.83 |

Beispiel 7

D,L-1-Benzyl-2,2-di(4-methoxyphenyl)-ethylendiamindichloro-platin(II)
Ausführung B (Reaktionszeit etwa 5 Stunden)
Ausbeute: 56%, blaßgelbes Pulver; F. >300°C (Zersetzung)
$C_{23}H_{26}Cl_2N_2O_2Pt$ (Molgewicht 628.5 g).
IR (KBr): 3260, 3160 $cm^{-1}$ (NH), 3090 (CH arom.), 2960, 2940 (CH aliph.), 1610, 1580 (NH), 330 (PtCl). -
$^1$H-NMR ($CDCl_3$, 250 MHz): $\delta$ = 6.87-8.31 (AA'BB', 4H, phenyl), 7.23-7.32 (m, 5H, phenyl), 6.89-7.17 (AA'BB', 4H, phenyl), 6.11, 6.19 (2m, 1H, $NH_2$), 4.83, 4,79 (2m, 2H, $NH_2$), 3.97, 3.87 (2m, 1H, $NH_2$), 3.89, 3.80 (2s, 6H, $OCH_3$), 3.26 (AB, 1H, CH-phenyl), 2.46 (m, 1H, CH-N), 2.22 (AB, 1H, CH-phenyl).

| Analysenwerte: | C | H | N |
|---|---|---|---|
| berechnet: | 43.95 | 4.17 | 4.46 |
| gefunden: | 44.49 | 4.54 | 4.19 |

Beispiel 8

D,L-1(4-Methoxybenzyl)-2,2-di(4-methoxyphenyl)-ethylendiamindichloro-platin(II)
Ausführung B

(Umfällung des Reaktionsproduktes wie bei Beispiel 5)
Ausbeute: 32%, blaßgelbes Pulver: F. >300° C (Zersetzung)
$C_{24}H_{28}Cl_2N_2O_3Pt$ (Molgewicht 658.5 g).
IR (KBr): 3240, 3180 $cm^{-1}$ (NH), 3100 (CH arom.), 2960, 2940 (CH aliph.), 1610, 1580 (NH), 330 (PtCl). - $^1$H-NMR ($CDCl_3$, 250MHz): $\delta$ = 6.86-8.32 (AA'BB', 4H, phenyl), 7.01-7.29 (AA'BB', 4H, phenyl), 6.80-7.07 (AA'BB', 4H, phenyl), 6.14, 6.22 (2m, 1H, $NH_2$), 4.82, 4.79 (2m, 2H, $NH_2$), 3.95, 4.02 (2m, 1H, $NH_2$), 3.89, 3.80, 3.20 (3s, 9H, $OCH_3$), 3.20 (AB, 1H, CH-phenyl), 2.46 (m, 1H, CH-N), 2.12 (AB, 1H, CH-phenyl).

Beispiel 9

D,L-1-Benzyl-1-phenyl-ethylendiamindichloro-platin(II)
Ausführung B
Ausbeute: 62%, blaßgelbes Pulver; F. >300° C (Zersetzung)
$C_{15}H_{18}Cl_2N_2Pt$ (Molgewicht 492,3 g).
IR (KBr): 3280, 3200 $cm^{-1}$ (NH), 3060 (CH arom.), 2960, 2920 (CH aliph.), 1610, 1580 (NH), 330 (PtCl). -$^1$H-NMR ([$D_7$]DMF, 250MHz): $\delta$ = 7.59-7.64, 7.38-7.48, 7.08-7.34, 6.92-6.96 (4m, 10H, phenyl), 5.52-5.57 (m, 4H, $NH_2$), 3.65 (AB, 1H, $^2J$ = 13.4, CH-phenyl), 3.53 (AB, 1H, CH-phenyl), 3.13-3.30 (m, 2H, $CH_2$).

| Analysenwerte: | C | H | N |
|---|---|---|---|
| berechnet: | 36.59 | 4.23 | 6.53 |
| gefunden: | 36.53 | 3.71 | 5.36 |

Beispiel 10

D,L-1-Benzyl-1-methyl-ethylendiamindichloro-platin(II)
Ausführung A
Ausbeute: 73%, blaßgelbes Pulver; F. >300° C (Zersetzung)
$C_{10}H_{16}Cl_2N_2Pt$ (Molgewicht 430.3 g).
IR (KBr): 3300, 3200 $cm^{-1}$ (NH), 3140 (CH arom.), 2960, 2940 (CH aliph.), 1610, 1580 (NH), 320 (PtCl). - $^1$H-NMR ([$D_7$]DMF, 250MHz): $\delta$ = 7.26-7.42 (m, 5H, phenyl), 5.37-6.07 (m, 2H, $NH_2$), 5.37, 5.41 (2m, 1H, $NH_2$), 5.02, 5.06 (2m, 1H, $NH_2$), 3.21 (s, 2H, $CH_2$-phenyl), 2.86-2.95, 2.53-2.58 (2m, 2H, von Lösungsmittelsignalen verdeckt, $CH_2$-N), 1.39 (s, 3H, $CH_3$).

| Analysenwerte: | C | H | N |
|---|---|---|---|
| berechnet: | 27.92 | 3.75 | 6.51 |
| gefunden: | 28.11 | 3.66 | 6.42 |

Beispiel 11

D,L-1-Benzyl-1-ethyl-ethylendiamindichloro-platin(II)
Ausführung A
Ausbeute: 54%, blaßgelbes Pulver; F. >300° C (Zersetzung)
$C_{11}H_{18}Cl_2N_2Pt$ (Molgewicht 444.3 g).
IR (KBr): 3200, 3280 $cm^{-1}$ (NH), 3140 (CH arom.), 2960, 2940 (CH aliph.), 1610, 1580 (NH), 320 (PtCl). $^1$H-NMR ([$D_7$]DMF, 250MHz): $\delta$ = 7.46-7.56, 7.33-7.37 (2m, 5H, phenyl), 5.54 (m, 2H, $NH_2$), 5.08, 5.11 (2m, 1H, $NH_2$), 4.72, 4.77 (2m, 1H, $NH_2$), 3.33 (AB, 1H, $^2J$ = 13.8 CH-phenyl), 3.10 (AB, 1H, CH-phenyl), 2.72-2.76 (von Lösungsmittelsignalen verdeckt, 2H, $CH_2$-N), 1,67-1.86 (m, 2H, $CH_2$), 1.06, 1.12 (t, 3H, $^3J$ = 7.6, $CH_3$).

| Analysenwerte: | C | H | N |
|---|---|---|---|
| berechnet: | 29.74 | 4.08 | 6.31 |
| gefunden: | 29.91 | 4.13 | 6.28 |

Beispiel 12

1,1-Dibenzyl-ethylendiamindichloro-platin(II)
Ausführung A (Diamin nach leichtem Erwärmen im Wasser löslich)
Ausbeute: 63%, blaßgelbes Pulver; F. >300° C (Zersetzung)
$C_{16}H_{20}Cl_2N_2Pt$ (Molgewicht 506.4 g).
IR (KBr): 3260, 3220 $cm^{-1}$(NH), 3120 (CH arom.), 2960 (CH aliph.), 1610, 1580 (NH), 330 (PtCl). - $^1$H-NMR ([D7]DMF, 250 MHz): $\delta$ = 7.44-7.59, 7.31-7.43 (2m, 10H, phenyl), 5.60 (m, 2H, $NH_2$), 4.63 (m, 2H, $NH_2$), 3.37 (AB, 2H, $^2J$ = 13.7, $CH_2$-phenyl), 3.10 (AB, 2H, $^2J$ = 13.7, $CH_2$-phenyl), 2.67 (m, 2H, $CH_2$-N).

| Analysenwerte: | C | H | N |
|---|---|---|---|
| berechnet: | 37.95 | 3.98 | 5.53 |
| gefunden: | 38.32 | 4.27 | 5.47 |

Beispiel 13

2-Amino-2-aminomethylindandichloro-platin(II)
Ausführung A

Ausbeute: 80%, blaßgelbes Pulver; F>300°C (Zersetzung)
$C_{10}H_{14}Cl_2N_2Pt$ (Molgewicht 428.2 g).

IR (KBr):

| $[cm^{-1}]$ | $\nu$(NH) sym. und asym. | CH arom. | CH aliph. | $\delta$(NH) | $\nu$(C=C) sym. und asym. | CH arom. out of plane | $\nu$PtCl |
|---|---|---|---|---|---|---|---|
| 13 | 3240s<br>3180m | 3050m | 2920w<br>2840 | 1590m<br>1580m | 1490m<br>1460m | 750s<br>800m | 310m |

$^1H$-NMR ([$D_7$]DMF, 250 MHz):

| δ [ppm] | | Zuordnung |
|---|---|---|
| 7.25-7.15 | (m, | phenyl), |
| 5.74 | (m, | $NH_2$), |
| 3.38 | (s, | $CH_2$-indan), |
| 2.92 | (m, | $CH_2$), |

| Analysenwerte: | C | H | N |
|---|---|---|---|
| berechnet: | 28.05 | 3.30 | 6.54 |
| gefunden: | 28.10 | 3.34 | 6.27 |

Beispiel 14

D,L-3-Aminomethyl-1,2,3,4-tetrahydroisochinolindichloro-platin(II)
Ausführung A
Ausbeute: 65%, blaßgelbes Pulver; F >300°C (Zersetzung) $C_{10}H_{14}Cl_2N_2Pt$ (Molgewicht 428.2 g).

IR (KBr):

| $[cm^{-1}]$ | ν(NH) sym. und asym. | CH arom. | CH aliph. | δ(NH) | ν(C=C) sym. und asym. | CH arom. out of plane | νPtCl |
|---|---|---|---|---|---|---|---|
| 14 | 3260m<br>3200m | 3120s | 2960w<br>2860w | 1590m<br>1580m | 1500m<br>1460m | 760s<br>710w | 310m<br>330m |

$^1$H-NMR ($[D_7]$DMF, 250 MHz):

| δ [ppm] | | Zuordnung |
|---|---|---|
| 7.11-7.22 | (m, | phenyl), |
| 6.33 | (m, | $NH_2$,NH), |
| 5.57 | (m, | $NH_2$,NH), |
| 4.68 | (AB-Syst. | $CH_2$, tetrahydroisochinolin) |
| 4.54 | (AB-Syst. | $CH_2$, tetrahydroisochinolin) |
| 4.03-4.14 | (m, | CH-tetrahydroisochinolin-N |
| 3.02-3.24 | (m, | CH-N/$CH_2$-tetrahydroiso-chinolin-N) |

| Analysenwerte: | C | H | N |
|---|---|---|---|
| berechnet: | 28.05 | 3.30 | 6.54 |
| gefunden: | 28.94 | 3.39 | 6.33 |

Beispiel 15

D,L-1-Amino-1-aminomethyltetralindichloro-platin(II)
Ausführung A

Ausbeute: 77%, gelbes Pulver
$C_{11}H_{16}Cl_2N_2Pt$ (Molgewicht 442.3 g)

IR (KBr): 3280, 3220 $cm^{-1}$ (NH), 3060 (CH arom.), 2970, 2920 (CH aliph.), 1590 (NH), 320 (PtCl). - $^1$H-NMR (DMF-$d_7$, 250 MHz): 7.30-7.14 (m, 4H, phenyl), 5.54 (m, 4H, $NH_2$), 3.75-3.68 (m, 2H, $CH_2$-phenyl),

2.71 (m, $CH_2$-N, von Lösungsmittelsignalen verdeckt), 2.29-1.28 (m, 4H, $CH_2$).

| Analysenwerte: | C | H | N |
|---|---|---|---|
| ber.: | 29.87 | 3.65 | 6.34 |
| gef.: | 30.22 | 3.64 | 6.29 |

Beispiel 16

D,L-4-Amino-4-aminomethlchromandichloro-platin(II)
Ausführung A

Ausbeute: 52 %, gelbes Pulver
$C_{10}H_{14}Cl_2N_2OPt$ (Molgewicht 444.2 g)
IR (KBr): 3240, 3200 $cm^{-1}$ (NH), 3070 (CH arom.), 2960 (CH aliph.), 1590 (NH), 320 (PtCl). - $^1$H-NMR (DMF-$d_7$, 250 MHz): 7.28-6.81 (m, 4H, phenyl), 5.79, 5.64 (2m, 4H, $NH_2$), 4.48-4.20 (m, 2H, $CH_2$-O), 3.37-3.25 (m, 2H, $CH_2$-N), 2.78-2.73 (m, 2H, $CH_2$, von Lösungsmittelsignalen verdeckt).

| Analysenwerte: | C | H | N |
|---|---|---|---|
| ber.: | 24.04 | 3.18 | 6.31 |
| gef.: | 27.39 | 3.20 | 6.09 |

Beispiel 17

D,L-2-Amino-2-aminomethyldecalindichloro-platin(II)
Ausführung A

Ausbeute: 62%, gelbes Pulver
$C_{11}H_{22}Cl_2N_2Pt$ (Molgewicht 448.3 g)
IR (KBr): 3200, 3140 $cm^{-1}$ (NH), 2920, 2860 (CH aliph.), 1580 (NH), 320 (PtCl). - $^1$H-NMR (DMF-$d_7$, 250 MHz): 5.40-5.02 (3m, 4H, $NH_2$), 2.78-2.73 (m, $CH_2$-N, 2H von Lösungsmittelsignalen verdeckt), 2.22-0.89 (m, 16H, CH, $CH_2$).

| Analysenwerte: | C | H | N |
|---|---|---|---|
| ber.: | 29.47 | 4.95 | 6.25 |
| gef.: | 30.28 | 5.07 | 6.18 |

Beispiel 18

D,L-1-(p-Methoxy)benzyl-1-methyl-ethylendiamindichloro-platin(II)
Ausführung A

Ausbeute: 68%, gelbe Nadeln, aus Acetonitril umkristallisiert
$C_{11}H_{18}Cl_2N_2OPt$ (Molgewicht 460.3 g)
IR (KBr): 3260, 3210 $cm^{-1}$ (NH), 3050 (CH arom.), 2960, 2930 (CH aliph.), 1610, 1570 (NH), 320 (PtCl). - $^1$H-NMR (DMF-$d_7$, 250 MHz): 7.32-6.39 (AA'BB', 4H, phenyl), 5.58-4.95 (4m, 4H, $NH_2$), 3.80 (s, 3H, $CH_3$-O), 3.15 (AB, 2H, $CH_2$-phenyl), 2.90-2.81 (m, $CH_2$-N, von Lösungsmittelsignalen verdeckt), 1.37 (s, 3H, $CH_3$)

| Analysenwerte: | C | H | N |
|---|---|---|---|
| ber.: | 28.70 | 3.94 | 6.09 |
| gef.: | 28.78 | 4.00 | 6.05 |

Beispiel 19

D,L-2-Amino-2-aminomethytetralindichloro-platin(II)
Ausführung A

Ausbeute: 39%, gelbe Nadeln
$C_{11}H_{16}Cl_2N_2Pt$ (Molgewicht 442.3 g)

IR (KBr): 3240, 3195 $cm^{-1}$ (NH), 3060 (CH arom.), 2950, 2910 (CH aliph.), 1580 (NH), 330 (PtCl). - $^1$H-NMR (DMF-$d_7$, 250 MHz): 7.14 (m, 4H, phenyl), 5.54-5.47 (m, 4H, $NH_2$), 3.26 (AB, 2H, $CH_2$-phenyl), 3.07-3.00 (m, 2H, $CH_2$-phenyl, von Lösungsmittelsignalen verdeckt), 2.45-2.40 (m, 2H, $CH_2$-N), 2.40-2.19 (m, 2H, $CH_2$).

| Analysenwerte: | C | H | N |
|---|---|---|---|
| ber.: | 29.87 | 3.65 | 6.34 |
| gef.: | 30.42 | 4.05 | 6.24 |

Beispiel 20

1-(p-Hydroxy)benzyl-1-methyl-ethylendiamindichloro-platin(II)
Ausführung A

Ausbeute: 57%, gelbe Nadeln
$C_{10}H_{16}Cl_2N_2OPt$ (Molgewicht 446.2 g)

IR (KBr): 3350 $cm^{-1}$ (OH), 3270, 3250 (NH), 3020 (CH arom.), 2960, 2920 (CH aliph.), 1585, 1620 (NH), 360 (PtCl). - $^1$H-NMR (DMF-$d_7$, 250 MHz): 9.51 (s, 1H, OH), 7.22-6.80 (AA'BB', 4H, phenyl), 5.55-4.89 (m, 4H, $NH_2$), 3.09 (AB, 2H, $CH_2$-Phenyl), 2.87-2.54 (m, 2H, $CH_2$-N, von Lösungsmittelsignalen verdeckt), 1.37 (s, 3H, $CH_3$).

Beispiel 21

Dichloro(1-Phenethyl-1-methyl-ethylendiamin)-Platin(II)

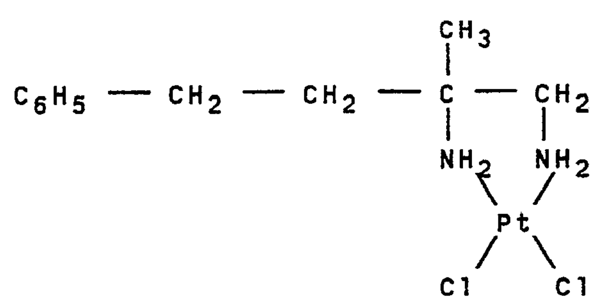

5 g (0,02 mol) 1-Phenethyl-1-methyl-ethylendiaminoxalat werden mit 4,5 KOH in 50 ml Wasser gelöst. Unter Rühren wird eine Lösung aus 8,26 g (0,02 mol) Kaliumtetrachloroplatinat in 50 ml $H_2O$ zugetropft und 2 Stunden bei 50°C gerührt.

Der Niederschlag wird abgesaugt, mit Wasser gewaschen und im Vakuum bei 40°C getrocknet.

Gelbes Pulver, F. 298°C (Zersetzung)
Ausbeute: 6,21 g

Der Glycolato-Komplex (Ersatz der 2 Cl durch Glycolsäure) wird zum Beispiel wie folgt erhalten: 12,5 g Chlorkomplex werden mit 9,56 g Silbernitrat in 80 ml Wasser und 8 ml Ethanol suspendiert. Unter Lichtausschluß wird mehrere Tage bei 45°C gerührt, der Niederschlag abgesaugt und das Filtrat mit 2,13 g (0,028 mol) Glycolsäure versetzt. Nach mehrtägigem Rühren bei Raumtemperatur wird am Rotationsverdampfer eingeengt, der Rückstand mit Diethylether gewaschen und im Vakuum getrocknet.
Weißes Pulver, F. 219 - 221°C (Zersetzung). Die Verbindung enhält 2 Mol Wasser.
Ausbeute: 9,5 g

Analog dem Glycolat-Komplex wird der D-Lactato-Komplex erhalten, indem anstelle der Glycolsäure 12,6 g (0,028 mol) D-Milchsäure 20 % eingesetzt werden. Gleiche Aufarbeitung.
F. 206 - 207°C (Zersetzung). Die Verbindung enthält 1 Mol Wasser.
Ausbeute: 9,8 g

Die Darstellung des L-Lactato-Komplexes erfolgt analog dem D-Lactato-Komplex mit 12,6 g (0,028 mol) L-Milchsäure 20 %.
Weißes Pulver, F. 203 - 204°C (Zersetzung)
Ausbeute: 10,5 g

Die Darstellung des Stearinato-Komplexes erfolgt analog dem Lactato-Komplex mit 14,34 (0,056 mol) Stearinsäure.
Gelbes Pulver, F. 228 °C (Zersetzung; Chiffre D 21 393).
Ausbeute: 8,3 g

Die Darstellung des Neodecanato-Komplexes erfolgt analog dem D-Lactato-Komplex mit 0,056 mol (9,6 g) Neodecansäure.
gelbes, pastöses Produkt (Chiffre D 21 392).
Ausbeute: 9,4 g; kein Schmelzpunkt

Der ω-Phenyloctadecanato(ω-Phenyl-stearlnato)-Komplex wird analog dem D-Lactato-Komplex mit 20,1 g (0,056 mol) ω-Phenyloctadecansäure (ω-Phenyl-stearinsäure) dargestellt.
gelblicher, leicht pastöser Feststoff (Chiffre D 21 394).
Ausbeute: 24 g; kein Schmelzpunkt

Der Sulfato-Komplex wird durch Umsetzung von 2 g (0,0045 mol) Chlorokomplex mit 1,25 g (0,004 mol) Silbersulfat in 800 ml Wasser/100 ml Ethanol-Mischung hergestellt. Die Reaktionszeit beträgt 4 Tage bei 45 - 50 °C. Nach Absaugen über Membranfilter werden 2,1 g weißes Produkt erhalten. (Chiffre D 21 407).
F.: 243 °C (Zersetzung).

Analog der Darstellung des D-Lactato-Komplexes werden 10 g Chlorokomplex (0,0225 mol) mit 5,9 g 0,045 mol N-Acetyl-L-alanin umgesetzt.
Ausbeute: 5,8 g weißes Pulver (Chiffre D 21 476).
F.: 225 °C (Zersetzung).
Der Komplex enthält noch 1 mol Wasser.

Der Nitrolotris(methylphosphonato)-Komplex wird analog dem D-Lactato-Komplex durch Umsetzung mit 0,056 mol (16,7 g) Nitrolotris-methylphosphonsäure dargestellt. Weißes Pulver F.: 254 °C (Chiffre D 21 569).
Ausbeute: 7,8 g
Der Komplex besitzt folgende Struktur:

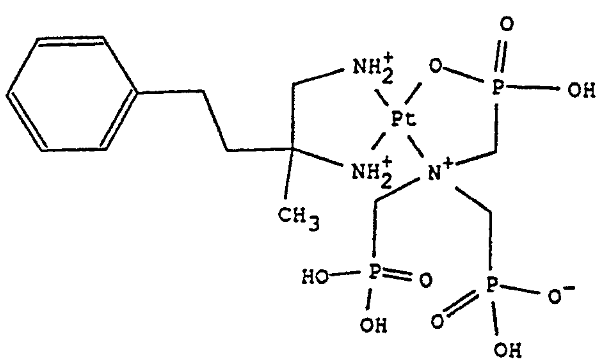

Beispiel 22
(Platin(IV)-Komplexe)

Dichloro-dihydroxy-(1-phenethyl-1-methylethylendiamin) -Platin(IV)

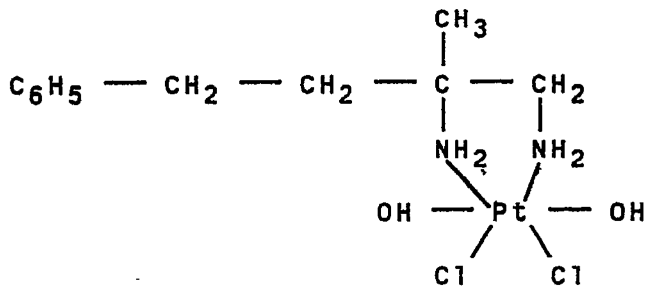

1g Dichloro(1-Phenethyl-1-methyl-ethylendiamin)-Platin(II) (0,002 Mol) werden in 30 ml Wasser suspendiert. Bei 70°C werden 10ml 35 %ige Wasserstoffperoxid-Lösung zugetropft. Die stark schäumende Suspension wird 4 Stunden bei 70°C gerührt, wobei eine klare Lösung entsteht.
Das überschüssige $H_2O_2$ wird vorsichtig mit 600 mg Platin-Aktivkohle zerstört (unter Rühren). Nach Filtration durch einen Membranfilter wird eingedampft und der Rückstand mit Diethylether gewaschen.
Fahlgelbes Pulver (Chiffre D 20 975) , F. 208 - 209°C (Zersetzung).
Ausbeute: 600 mg

Dihydroxy-distearinato(1-phenethyl-1-methyl-ethylendiamin)Platin(IV)

Der entsprechende Distearinato-Komplex (Ersatz der beiden Cl durch 2 Stearinsäureanionen) wird wie folgt erhalten:
1 g Stearinato-Platin(II)-Komplex (Chiffre D 21 393) wird in 30 ml $H_2O$ suspendiert und bei 70 °C 10 ml 35 % $H_2O_2$-Lösung vorsichtig zugetropft (starkes Schäumen). Nach 5-stündigem Rühren bei 70°C wird mit 600 mg Platin-Aktivkohle versetzt, über einen Membranfilter filtriert, eingedampft und der Rückstand mit Ether gewaschen.
Gelbes Pulver (Chiffre D 21 406), F. 200 - 202 °C (Zersetzung).
Ausbeute: 400 mg

Dihydroxy-dineodecanato (1-phenethyl-1-methyl-ethylendiamin)Platin(IV)

1 g (0,001 mol) Neodecanato-Platin(II)-Komplex (Substanz D 21 392) werden analog der Dihydroxydistearinato-Verbindung (siehe oben) mit 10 mg 35 % $H_2O_2$-Lösung oxidiert.
gelbes Produkt, das zu klebriger Masse erstarrt
Ausbeute: 1,0 g (Chiffre D 21 423);
kein Schmelzpunkt.

Beispiel 23

(Weitere Beispiele für den Austausch der Gruppe X)

Darstellung von Lactat-Komplexen

Die für diese Austauschreaktion verwendete Milchsäure muß frisch destilliert sein, da sich bei längerer Lagerung polymere Ester ausbilden können.

Der Platinkomplex mit dem Anion X (zum Beispiel X = Cl) wird zuerst zum Beispiel auf folgende Weise in den entsprechenden Diamin-diaqua-platin(II)-Komplex überführt.

5 mMol Platin-Komplex (zum Beispiel Chloro-Komplex) werden in ca. 25 ml $H_2O$ suspendiert. Dazu wird der Ansatz abwechselnd eine Stunde in ein Ultraschallbad gebracht und eine Stunde gerührt, wobei der an der Kolbenwand sich absetzende Komplex immer wieder in die Suspension zurück gespült wird. Nach dreibis viermaliger Wiederholung der Prozedur ist eine ausreichende Suspension gewährleistet. Unter Lichtausschluß wird dann eine Lösung von 10 mMol (1.86 g) $AgNO_3$ in ca. 5 ml $H_2O$ zugegeben. Nach einer Woche Rühren des Ansatzes wird das ausgefallene AgCl als hellgrauer Niederschlag über eine Membranfilterfritte abfiltriert. Der Diaquakomplex bleibt gelöst.

D,L-1-Benzyl-2,2-dimethyl-ethylendiamin-L-lactato-platin(II)

5 mMol (0.45 g) L-(+)-Milchsäure werden in 10 ml destilliertem Wasser gelöst, um als Vorlage für die Ionenaustauschersäule zu dienen.

25 g stark basischer Ionentauscher (Fa. Merck; Ionenaustauscher III; Austauschkapazität 4 mVal/g; das heißt 25 g = 100 mVal, also 10 facher Überschuß gegenüber dem Platin-Komplex) werden in eine Chromatographiesäule gefüllt. Dabei sollte das Verhältnis von Säulendurchmesser zu Säulenlänge 1:4 bis 1:10 sein.

Um das Austauscherharz mit $OH^-$ zu belegen, wird zunächst achtmal mit dem Harzvolumen an 2N NaOH gespült. Der pH-Wert des Auslaufes wird dann durch Nachwaschen mit $H_2O$ auf ca. pH 9 eingestellt.

Auf die so vorbereitete Säule wird schließlich das Filtrat des D,L-1-Benzyl-2,2-dimethyl-ethylendiamindiaquakomplexes gebracht und mit einer Durchlaufgeschwindigkeit von ca. 2 cm/Minute in die gerührte Vorlage getropft.

Nach vollständiger Zugabe läßt man noch 3-4 Stunden bei 40° C rühren.

Zur Isolierung der Komplexe muß zunächst das Wasser ganz abgezogen werden. Der glasige Rückstand wird dann mit ca. 5 ml Ethanol aufgenommen und die Lösung mit 100 ml Ether versetzt. Dabei erhält man einen farblosen, flockigen Niederschlag. Beim Abfiltrieren des Niederschlages unter Stickstoff-Schutzgas sollte der Filterkuchen möglichst schnell trocken gesaugt werden, da er sich, solange er naß ist, an Luft schnell braun verfärbt und zu zerfließen beginnt.

Ausbeute; 67%, (bezüglich des Chloro-Komplexes) farbloses Pulver

$C_{14}H_{22}N_2O_3Pt$ (Molgewicht 461.4 g).

IR (KBr): 3200, 3080 $cm^{-1}$ (NH), 3060 (CH arom.), 2980 (CH aliph.), 1680, 1610 (C=O), 380 (PtO).-

$^1$H-NMR ($D_2O$, 250 MHz): $\delta$ = 7.25-7.34 (m, 5H, phenyl), 4.35, 4.02, 3.57 (3m, 1H, CH), 2.83-2.93 (m, 1H, CH-N), 2.46-2.72 (m, 2H, $CH_2$-phenyl), 1.09-1.46 (m, 9H, $CH_3$).

| Analysenwerte: | C | H | N |
|---|---|---|---|
| berechnet: | 36.50 | 4.80 | 6.07 |
| gefunden: | 36.34 | 5.04 | 5.84 |

Analog werden die folgenden Lactato-Komplexe erhalten.

D,L-1-Benzyl-1-methyl-ethylendiamin-L-lactato-platin(II)

Ausbeute: 32%, (bezüglich des Chloro-Komplexes) farbloses Pulver

$C_{13}H_{20}N_2O_3Pt$ (Molgewicht 447.4 g).

IR (KBr): 3200, 3100 $cm^{-1}$ (NH), 3040 (CH arom.), 2980, 2940 (CH aliph.), 1750, 1610 (C=O), 370 $cm^{-1}$ (PtO).-

$^1$H-NMR ($D_2O$, 250MHz): $\delta$ = 7.23-7.32 (m, 5H, phenyl), 3.98-4.02 (m, 1H, CH), 2.83-3.55, 2.38-2.62 (2m, 4H, $CH_2$-phenyl, $CH_2$-N), 1.14-1.35 (m, 6H, $CH_3$).

| Analysenwerte: | C | H | N |
|---|---|---|---|
| berechnet: | 34.90 | 4.51 | 6.26 |
| gefunden: | 34.79 | 5.04 | 6.01 |

D,L-1-Benzyl-1-ethyl-ethylendiamln-L-lactato-platin(II)

Ausbeute: 54%, (bezüglich des Chloro-Komplexes), farbloses Pulver

$C_{14}H_{22}N_2O_3Pt$ (Molgewicht 461.4 g).

IR (KBr): 3200, 3080 $cm^{-1}$ (NH), 3040 (CH arom.), 2980, 2940 (CH aliph.), 1730, 1610 (C=O), 760, 700 (PtO).-

$^1$H-NMR ($D_2O$, 250MHz): $\delta$ = 7.21 (m, 5H, phenyl), 4.34, 4.10, 3.99, 3.56 (4m, 1H, CH), 2.88-2.99, 2.41-2.55

(2m, 4H, $CH_2$-phenyl, $CH_2$-N), 1.46-1.76 (m, 2H, $CH_2$), 1.34, 1.22, 1.17, 1.10 (4d, 3H, $^3J$ = 6.9, CH), 0.91-0.96 (m, 3H, $CH_3$).

| Analysenwerte: | C | H | N |
|---|---|---|---|
| berechnet: | 36.44 | 4.81 | 6.07 |
| gefunden: | 35.73 | 4.99 | 5.55 |

1.1-Dibenzyl-ethylendiamin-L-lactato-platin(II)
Ausbeute: 32%, (bezüglich des Chloro-Komplexes), farbloses Pulver
$C_{19}H_{24}N_2O_3Pt$ (Molgewicht 523.5 g).
IR (KBr): 3200, 3080 $cm^{-1}$ (NH), 3040 (CH arom.), 2980 (CH aliph.), 1730, 1610 (C=O), 370 (PtO).

Analysenwerte: berechnet für den Komplex mit 2 $H_2O$

| | C | H | N |
|---|---|---|---|
| berechnet: | 40.78 | 5.04 | 5.00 |
| gefunden: | 40.60 | 4.92 | 5.00 |

D,L-1-Benzyl-1-ethyl-ethylendiaminglycolato-platin(II)

Ausbeute: 58 %, farbloses Pulver
$C_{13}H_{20}N_2O_3Pt$ (Molgewicht 447.4 g)
IR (KBr): 3220, 3110 $cm^{-1}$ (NH), 3040 (CH arom.), 2980, 2950 (CH aliph.), 1640 (C0), 370 (Pt0). - $^1$H-NMR ($D_20$, 250 MHz): 7.31 (m, 5H, phenyl), 3.94 (AB, 2H, $CH_2$-0), 3.04-2.83 (m, 2H, $CH_2$-phenyl), 2.49-2.47 (m, 2H, $CH_2$-N), 1.85-1.51 (m, 2H, $CH_2$), 1.08, 0.96 (2t, 3H, $CH_3$).

| Analysenwerte: | C | H | N |
|---|---|---|---|
| berechnet: | 34.90 | 4.51 | 6.26 |
| gefunden: | 34.44 | 4.82 | 5.35 |

D,L-1-Benzyl-1-ethyl-ethylendiaminmandelato-platin(II)

Ausbeute: 47 %, farbloses Pulver
$C_{19}H_{24}N_2O_3Pt$ (Molgewicht 523.5 g)
IR (KBr): 3220, 3100 $cm^{-1}$ (NH), 3060, 3040 (CH arom.), 2980, 2940 (CH aliph.), 1640 (C0), 360 (Pt0). - $^1$H-NMR ($CD_30D$, 250 MHz): 7.72, 7.31 (2m, 10H, phenyl), 4.86, 4.88 (2s, 1H, CH-phenyl), 3.11-2.96 (m, 2H, $CH_2$-phenyl), 2.51-2.43 (m, 2H, $CH_2$-N), 1.89-1.55 (m, 2H, $CH_2$), 1.07, 1.06 (2t, 3H, $CH_3$).

| Analysenwerte: | C | H | N |
|---|---|---|---|
| berechnet: | 43.59 | 4.63 | 5.35 |
| gefunden: | 43.56 | 4.88 | 5.49 |

2-Amino-2-aminomethylindan-L-lactato-platin(II)
Ausbeute: 36%, (bezüglich des Chloro-Komplexes) farbloses Pulver
$C_{13}H_{18}N_2O_3Pt$: (Molgewicht 445.4 g).

IR (KBr): 3280, 3220 $cm^{-1}$ (NH), 3060 (CH aromatisch), 2970, 2920 (CH aliphatisch, 1590(NH), 320(PtCl).

Analysenwerte: berechnet für den Komplex mit 2 $H_2O$

| | C | H | N |
|---|---|---|---|
| berechnet: | 32.40 | 4.61 | 5.81 |
| gefunden: | 31.95 | 4.43 | 5.44 |

Der Austausch der Gruppe X unter Verwendung anderer anionischer-Gruppen bzw. Säuren erfolgt beispielsweise in analoger Weise wie vorstehend beschrieben ist.

Beispiel 24

(Beispiele für die Cyclodextrin - Platin-Komplex - Präparationen)

Präparation von D,L-1-Benzyl-2,2-dimethyl-ethylendiamindichloro-platin(II) mit α-Cyclodextrin.

$1x10^{-5}$ Mol (4.4 mg) des zuvor genannten Platin-Komplexes werden in ca. 50 ml Ethanol unter leichtem Erwärmen (30° C) gelöst.
Die doppelt äquivalente Menge α-Cyclodextrin, also $2x10^{-5}$ Mol (19 mg) werden in ca. 10 ml destilliertem Wasser gelöst.
Beide Lösungen werden vereint, und das Lösungsmittel wird am Rotationsverdampfer abgezogen.
Der glasige Rückstand wird am Hochvakuum ($10^{-4}$ Torr) getrocknet und beispielsweise für den Test an der P 388 Maus-Leukämie in 10 ml Lösungsmittelgemisch aus Polyethylenglykol 400 und 1.8 %iger Kochsalzlösung (1:1) im Ultraschallbad gelöst.

Beispiel 25

(Beispiel für die Herstellung von Polyvinylpyrrolidon-Kopräzipitaten)

$1x10^{-5}$ Mol D,L-1-Benzyl-1-methyl-ethylendiamindichloro-platin(II) werden in ca. 50 ml Ethanol unter Erwärmen (30° C) gelöst.
56 mg Polyvinylpyrrolidon-10 (Durchschnitts-Molekulargewicht 10.000) werden in 10 ml Ethanol gelöst und zur Lösung des Platin-Komplexes gegeben. Nach dem Abziehen des Lösungsmittels erhält man einen gelben glasigen Rückstand, der am Hochvakuum getrocknet wird. Für die Herstellung der Applikationslösung wird dieses Kopräzipitat beispielsweise im Ultraschallbad in 10 ml des Lösungsmittelgemisches Polyethylenglykol 400/1.8%ige Kochsalzlösung (1:1) gelöst.
Herstellung von C2 - substituierten Benzyl-ethylendiamin-Liganden der Formel

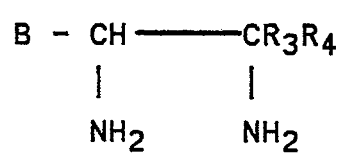

sowie von cyclischen Aminliganden sowie von $C_1$-substituierten Benzyl-ethylendiamin-Liganden, die am Benzylrest substituiert sind.

Beispiel 26

D,L-1-Benzyl-2,2-dimethylethylendiamin

9 mMol (1.8 g) Amino-Azid D,L-1-Phenyl-2-amino-3-azido-3-methylbutan werden in ca. 30 ml Ether gelöst und unter Eiskühlung zu einer Suspension von 20 mMol (0,76 g) $LiAlH_4$ in 30 ml Ether getropft. Nach

4-5 stündigem Kochen unter Rückfluß läßt man abkühlen und hydrolysiert bei 0-5° C mit feuchtem Ether und wenig Wasser. Das schlammartige Hydrolyseprodukt wird abgesaugt und das Filtrat über $Na_2SO_4$ getrocknet.
Nach dem Abziehen des Lösungsmittels wird der Rückstand im Hochvakuum ($10^{-4}$ Torr) bei ca. 90° c kugelrohrdestilliert.

Der Rückstand der Hydrolyse wird in einer Soxhlet-Apparatur mit 100 ml $CH_2Cl_2$ über Nacht extrahiert. Der Extrakt wird nach dem Trocknen vom Lösungsmittel befreit und der Rückstand ebenfalls im Hochvakuum bei 90° C destilliert.
Ausbeute: 50 % farbloses Öl
IR: siehe Tabelle 1

Beispiel 27

D,L-1-(4-Chlorbenzyl)-2,2-dimethylethylendiamin
Durchführung: analog Beispiel 26 unter Verwendung von 9 mMol (1.8 g) D,L-1-(4-Chlorphenyl)-2-amino-3-azido-3-methylbutan.Kugelrohrdestillation im Hochvakuum ($10^{-5}$ Torr) bei 120° C.
Ausbeute: 54 % farbloses Öl
IR: siehe Tabelle 1

Beispiel 28

D,L-1-Benzyl-2,2-diethylethylendiamin
Durchführung: analog Beispiel 26 unter Verwendung von 9 mMol (2.1 g) D,L-1-Phenyl-2-amino-3-azido-3-ethyl-n-pentan. Kugelrohrdestillation im Hochvakuum ($10^{-4}$ Torr) bei 90° C.
Ausbeute: 90 % farbloses Öl
IR: siehe Tabelle 1

Beispiel 29

D,L-1-Benzyl-2,2-di-n-pentylethylendiamin
Durchführung: analog Beispiel 26 unter Verwendung von 9 mMol (2.8 g) D,L-1-Phenyl-2-amino-3-azido-3-n-pentyl-n-octan.Kugelrohrdestillation im Hochvakuum ($10^{-5}$ Torr) bei 140° C.
Ausbeute: 90 % farbloses Öl
IR: siehe Tabelle 1

Beispiel 30

D,L-1-Benzyl-2,2-dicyclohexylethylendiamin
Durchführung: analog Beispiel 26 unter Verwendung von 9 mMol (2.8 g) D,L-1,1-Dicyclohexyl-1-azido-2-amino-3-phenylpropan.
IR: siehe Tabelle 1
Darstellung des Dihydrochlorids:
1.5 g Diamin werden in 10 ml Ether gelöst, und die Lösung wird auf -70° C abgekühlt. Nach kurzem Einleiten von HCl-Gas erhält man einen gelblichen Niederschlag, der abfiltriert, mit Ether gewaschen und getrocknet wird. Das Dihydrochlorid ist ein blaßgelbes, hygroskopisches Pulver.

Beispiel 31

D,L-1-Benzyl-2,2-diphenylethylendiamin
Durchführung analog Beispiel 26 unter Verwendung von 9 mMol (3.0 g) D,L-1,1-Diphenyl-1-azido-2-amino-3-phenylpropan. Kugelrohrdestillation im Hochvakuum ($10^{-4}$ Torr) bei 130° C.
Ausbeute: 67 % braunes, halbfestes Öl.
IR: siehe Tabelle 1

Beispiel 32

D,L-1-Benzyl-2,2-di(4-methoxyphenyl)ethylendiamin
Duchführung: analog Beispiel 26 unter Verwendung von 9 mMol (83.2 g) D,L-1,1-Di(4-methoxyphenyl)-1-azido-2-amino-3-phenylpropan. Kugelrohrdestillation im Hochvakuum ($10^{-4}$ Torr) bei 200° C.
Ausbeute: 30 % halbfestes Öl
IR: siehe Tabelle 1

Beispiel 33

D,L-1-(4-Methoxybenzyl)-2,2-di(4-methoxyphenyl) ethylendiamin
Durchführung: analog Beispiel 26 unter Verwendung von 9 mMol (3.8 g) D,L-1,1-Di(4-methoxyphenyl)-1-azido-2-amino-3-(4-methoxyphenyl)propan. Reinigung durch Chromatographie über eine Kieselgelsäule (50 cm x 5 cm). Die Substanz wird in Toluol/Ether (1:1) aufgetragen. Mit diesem Elutionsmittel wird eine 1. Zone abgetrennt, die verworfen wird. Das Diamin wird schließlich mit Methanol von der Säule gewaschen.
Ausbeute: 20 % gelbes, halbfestes Öl
IR: siehe Tabelle 1

Beispiel 34

D,L-1-Amino-1-aminomethyltetralin

69.7 mMol (2.6 g) $LiAlH_4$ werden in 70 ml Tetrahydrofuran suspendiert. Zu dieser Suspension werden 34.8 mMol (6.0 g) D,L-1-Amino-1-cyanotetralin in 30 ml Tetrahydrofuran bei Eiskühlung zugetropft. Nach dem Entfernen des Eisbades wird über Nacht refluxiert. Die Hydrolyse erfolgt durch Zutropfen von 20 ml Wasser unter Eiskühlung. Das schlammige Hydrolyseprodukt wird abfiltriert und über Nacht mit 250 ml Tetrahydrofuran in einer Soxhlet-Apparatur extrahiert. Das Filtrat und die Lösung der Extraktion werden vereinigt, und das Lösungsmittel wird abgezogen. Man erhält ein braunes Öl, das im Hochvakuum ($10^{-4}$Torr) kugelrohrdestilliert wird.
Bei 60°C destilliert das Nebenprodukt über. Das Diamin erhält man bei einer Temperatur von 90°C.
Ausbeute: 18 %, farbloses Öl
$C_{11}H_{16}N_2$ (Molgewicht 176.3 g)
IR: siehe Tabelle 1

Beispiel 35

D,L-4-Amino-4-aminomethylchroman
Durchführung: analog Beispiel 26 unter Verwendung von 20.1 mMol (3.5 g) D,L-4-Amino-4-cyanochroman.
Kugelrohrdestillation ($10^{-4}$Torr):

1. Fraktion bei 70°C (Nebenprodukt)
2. Fraktion bei 100°C (Diamin)

Ausbeute: 27%, gelbes Öl
$C_{10}H_{14}N_2O$ (Molgewicht 178.2 g)
IR: siehe Tabelle 1

Beispiel 36

D,L-2-Amino-2-aminomethyldecalin
Durchführung: analog Beispiel 26 unter Verwendung von 62.8 mMol (11.2 g) D,L-2-Amino-2-cyanodecalin.
Kugelrohrdestillation ($10^{-4}$ Torr):

1. Fraktion bei 60°C (Nebenprodukt)
2. Fraktion bei 100°C (Diamin)

Ausbeute: 10%, farbloses Öl
$C_{11}H_{22}N_2$ (Molgewicht 182.3 g)
IR: siehe Tabelle 1

Beispiel 37

D,L-1-(p-Methoxy)benzyl-1-methyl-ethylendiamin
Durchführung: analog Beispiel 26 unter Verwendung von 33.6 mMol (6.4 g) D,L-1-(p-Methoxy)phenyl-2-amino-2-cyanopropan.
Kugelrohrdestillation ($10^{-4}$ Torr):

1. Fraktion bei 65°C (Nebenprodukt)
2. Fraktion bei 90°C (Diamin)

Ausbeute: 15 %, farbloses Öl
$C_{11}H_{18}N_2O$ (Molgewicht 194.3 g)

IR: siehe Tabelle 1

Beispiel 38

2-Amino-2-aminomethyltetralin

Durchführung: analog Beispiel 26 unter Verwendung von 31.0 mMol (5.9 g) Tetralin-2-amino-2-carbonsäureamid.
Kugelrohrdestillation:
Siedepunkt: 110°C ($10^{-4}$ Torr)

Ausbeute: 45 %, farbloses Öl
$C_{11}H_{11}N_2$ (Molgewicht 176.3 g)

IR: siehe Tabelle 1

Beispiel 39

1-(p-Hydroxy)benzyl-1-methyl-ethylendiamin

7.21 mMol (1.4 g) 1-(p-Methoxy)benzyl-1-methyl-ethylendiamin werden in 150 ml Methylenchlorid gelöst 150 ml Methylenchlorid gelöst und auf -78°C abgekühlt. Anschließend läßt man bei der gleichen Temperatur 43.21 mMol (4.1 ml) $BBr_3$ zutropfen, eine Stunde bei -78°C und dann 20 Stunden bei 20°C rühren. Bei 0°C wird mit 30 ml Methanol hydrolysiert, wobei ein Teil des Produktes auskristallisiert. Nach Abziehen des Lösungsmittels erhält man das Produkt quantitativ als farblosen Feststoff.

Ausbeute: quantitativ, farbloser Feststoff
$C_{10}H_{16}N_2O$ (Molgewicht 180.2 g)

IR: siehe Tabelle 1

Tabelle 1

IR - Daten der C2-substituierten Benzylethylendiamine (Film) [$cm^{-1}$]

| Beispiel Nr. | ν(NH) sym. und asym. | CH arom. | CH aliph. | δ(NH) | ν(C=C) sym. und asym. | CH arom. out of plane |
|---|---|---|---|---|---|---|
| 26 | 3390m<br>3300m | 3020m | 2980s | 1600s | 1500s<br>1450s | 700s<br>750s |
| 27 | 3360m<br>3280m | 3040w | 2980s<br>2960m | 1600s | 1500s<br>1450m | 840m<br>810s |
| 28 | 3380m<br>3320m | 3040s | 2980s<br>2960s | 1610s | 1500s<br>1460s | 700s<br>750s |
| 29 | 3400m<br>3320m | 3040s | 2980s<br>2960s | 1610s | 1500s<br>1460s | 710s<br>750s |
| 30 | 3340m | 3040m | 2940s<br>2860s | 1660m | 1500m<br>1460s | 700s<br>730m |
| 31 | 3380m<br>3300m | 3040s<br>3060s | 2920s<br>2860m | 1610s | 1500s<br>1450s | 700s<br>750s |
| 32 | 3400m<br>3340m | 3020s<br>3040s | 2960s<br>2920s | 1620s<br>1620s | 1510s<br>1450s | 700s<br>750s |
| 33 | 3360b | 3020m<br>3040m | 2960s<br>2920m | 1620s<br>1580m | 1510s<br>1470m | 830s |

Tabelle 1 (Fortsetzung)

| | ν(NH) sym u. asym. | ν(CH) arom. | ν(CH) aliph. | δ(NH) | ν(C=C) | δ(CH) out of plane |
|---|---|---|---|---|---|---|
| 34 | 3370 sb<br>3300 sb | 3060 m<br>3020 m | 2930 s<br>2860 s | 1600 s | 1480 s<br>1440 s | 740 s |
| 35 | 3380 sb<br>3300 sb | 3080 m<br>3040 m | 2950 s<br>2880 s | 1620 s<br>1580 s | 1500 s<br>1460 s | 770 s |
| 36 | 3280 sb | | 2900 s<br>2860 s | 1600 s | | |
| 37 | 3360 sb<br>3280 sb | 3040 w<br>3000 w | 2910 s<br>2820 s | 1615 s<br>1590 s | 1510 s<br>1565 s | 840 s |
| 38 | 3100-<br>3400 sb | 3020 w | 2920 s<br>2850 m | 1600 s | 1500 s<br>1460 s | 740 s |
| 39<br>IR in<br>KBr | 3360 sb<br>3310 sb<br>3530 (OH) sb | 3030 w<br>3010 w | 2920 w<br>2850 w | 1620 s<br>1585 s | 1525 s | 835 s |

Herstellung von C1-substituierten Benzyl-ethylendiamin-Liganden der Formel

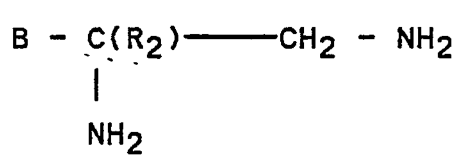

Beispiel 40

D,L-1-Benzyl-1-phenylethylendiamin

Durchführung: analog Beispiel 26 unter Verwendung von 9 mMol (2.3 g) D,L-1,2-Diphenyl-2-azido-3-aminopropan.

Kugelrohrdestillation im Hochvakuum ($10^{-4}$ Torr) bei 180° C.

Ausbeute: 83 % farbloses Öl

IR: siehe Tabelle 2

Beispiel 41

D,L-1-Benzyl-1-methylethylendiamin

0.4 Mol (15.2 g) $LiAlH_4$ werden als Suspension in ca. 100 ml Tetrahydrofuran vorgelegt. 0.1 Mol (16.02 g) D,L-1-Phenyl-2-amino-2-cyanopropan werden in 150 ml Tetrahydrofuran gelöst und unter Eiskühlung über einen Tropftrichter zur $LiAlH_4$-Suspension getropft. Nach vollständiger Zugabe wird das Eisbad entfernt und 15 Stunden am Rückfluß gekocht.

Zur Hydrolyse werden unter Eiskühlung 1.6 Mol (28.8 ml) destilliertes Wasser vorsichtig unter kräftigem Rühren zugetropft.

Das schlammige Hydrolyseprodukt wird abfiltriert und in einer Soxhlet-Apparatur 15 Stunden mit 250 ml Tetrahydrofuran extrahiert. Der Rückstand, den man nach Abziehen des Lösungsmittels erhält, wird im Hochvakuum ($10^{-4}$ Torr) kugelrohrdestilliert.

Bei einer Übergangstemperatur von 70° C kann die Fraktion des Nebenproduktes vollständig abdestilliert werden.

Das Diamin wird schließlich bei 100° C überdestilliert.

Ausbeute: 42 % farbloses Öl

IR: siehe Tabelle 2

Beispiel 42

D,L-1-Benzyl-1-ethylethylendiamin

Durchführung: analog Beispiel 41 unter Verwendung von 0.1 Mol (17.4 g) D,L-1-Phenyl-2-amino-2-cyanobutan.

Kugelrohrdestillation im Hochvakuum ($10^{-4}$ Torr):

1. Fraktion bei 60° C (Nebenprodukt)
2. Fraktion bei 95° C (Diamin)

Ausbeute: 28%, farbloses Öl

IR: siehe Tabelle 2

Beispiel 43

1,1-Dibenzylethylendiamin

Durchführung: analog Beispiel 41 unter Verwendung von 0,1 Mol (23.6 g) 1,3-Diphenyl-2-amino-2-cyanopropan

Kugelrohrdestillation im Hochvakuum ($10^{-4}$ Torr):

1. Fraktion: 100° C (Nebenprodukt)
2. Fraktion: 150° C (Diamin)

Ausbeute: 32 % leicht gelbliches Öl

IR: siehe Tabelle 2

Tabelle 2

IR - Daten der Cl-substituierten Benzylethylendiamine (Film) [$cm^{-1}$]

| Beispiel Nr. | ν(NH) sym. und asym. | CH arom. | CH aliph. | δ(NH) | ν(C=C) sym. und asym. | CH arom. out of plane |
|---|---|---|---|---|---|---|
| 40 | 3390m<br>3300m | 3040 | 2920m<br>2860m | 1610s<br>1590w | 1500s<br>1460s | 710s<br>760s |
| 41 | 3290m<br>3300m | 3020s | 2920s<br>2860m | 1610s<br>1590m | 1500s<br>1460s | 710s<br>760m |
| 42 | 3390m<br>3300m | 3020s | 2920s<br>2860m | 1610s<br>1590m | 1500s<br>1460s | 710s<br>760m |
| 43 | 3390m<br>3300 | 3040s | 2920s<br>2860s | 1610s<br>1590m | 1500s<br>1460s | 710s<br>760m |

Herstellung von cyclischen Benzylethylendiaminen

Beispiel 44

2-Amino-2-aminomethylindan

Durchführung: analog Beispiel 41 unter Verwendung von 0.1 Mol (15.8 g) 2-Amino-2-cyanoindan.
Kugelrohrdestillation im Hochvakuum ($10^{-4}$ Torr) bei 150° C
Ausbeute: 46 % farbloser Feststoff

IR (KBr): [$cm^{-1}$] siehe folgende Tabelle:

| ν(NH) sym. und asym. | CH arom. | CH aliph. | δ(NH) | ν(C=C) sym. und asym. | CH arom. out of plane |
|---|---|---|---|---|---|
| 3320m<br>3240m | 3080m<br>3020m | 2900s<br>2840m | 1610m<br>1580m | 1490s<br>1440s | 750s<br>710w |

Beispiele für galenische Zubereitungen

Beispiel für Kapseln:

1 kg Verbindung gemäß Beispiel 21 in Form des L-Milchsäure-Komplexes, 625 g mikrokristalline Cellulose und 11 g hochdisperses Siliciumdioxid werden durch ein Sieb der Maschenweite 0,8 mm gegeben und homogenisiert. Anschließend wird dieser Mischung 39 g Magnesiumstearat (gesiebt 0,8 mm) hinzugefügt und nochmals 1 Minute gemischt.
Zur Herstellung der Kapseln wird die Kapselmasse in bekannter Weise auf einer mit Formatteilen der Größe 00 ausgerüsteten Kapselmaschine in Hartgelatinekapseln der Größe 00 abgefüllt. Die Füllmenge pro Kapsel beträgt 670 mg entsprechend 400 mg Wirkstoff.

Beispiel für ein Lyophilisat mit Gerüstbildner:

In 900 ml Wasser für Injektionszwecke werden 20 g Verbindung gemäß Beispiel 21 und 94 g Mannit unter Rühren gelöst. Anschließend wird mit Wasser für Injektionszwecke auf 1 Liter aufgefüllt.
Diese Lösung wird unter aseptischen Bedingungen über ein Membranfilter von 0,2 µm Porenweite sterilfiltriert und zu 2 ml in 10 ml Injektionsflaschen der hydrolytischen Klasse I abgefüllt. Die Flaschen werden mit einem Gefriertrocknungsstopfen versehen und in einer geeigneten Anlage lyophilisiert. Nach der Trocknung begast man mit sterilem, getrocknetem Stickstoff und verschließt die Flaschen in der Anlage. Die Stopfen werden durch eine Bördelkappe gesichert.
Für die intravenöse Anwendung wird das Lyophilisat in 4 ml Wasser für Injektionszwecke aufgelöst.
1 Injektionsflasche enthält 40 mg Wirkstoff, 1 ml Lösung enthält 10 mg Wirkstoff.

Beispiel für ein Lyophilisat ohne Gerüstbildner:

In 3900 ml Wasser für Injektionszwecke werden 20 g Verbindung gemäß Beispiel 21 unter Rühren gelöst. Anschließend wird mit Wasser für Injektionszwecke auf 4 Liter aufgefüllt.
Diese Lösung wird unter aseptischen Bedingungen über ein Membranfilter von 0,22 µm Porenweite sterilfiltriert und zu 8 ml in 30 ml Injektionsflaschen der hydrolytischen Klasse I abgefüllt. Die Flaschen werden mit einem Gefriertrocknungsstopfen versehen und in einer geeigneten Anlage lyophilisiert. Nach der Trocknung begast man mit sterilem, getrocknetem Stickstoff und verschließt die Flaschen in der Anlage. Die Stopfen werden durch eine Bördelkappe gesichert.
Für die intravenöse Anwendung wird das Lyophilisat in 8 ml Wasser für Injektionszwecke aufgelöst.
1 Injektionsflasche enthält 40 mg Wirkstoff, 1 ml Lösung enthält 5 mg Wirkstoff.

Beispiel für eine Phospholipiddispersion:

20 g Verbindung gemäß Beispiel 21, wobei an Stelle der zwei Cl zwei Stearinsäureanionen vorliegen,

werden in 4 kg $CH_2Cl_2$ unter Rühren gelöst. Anschließend werden 399,8 g Sojalecithin und 0,2 g α Tocopherol zugegeben und unter Rühren gelöst. Das organische Lösungsmittel wird im Vakuum entfernt (zum Beispiel durch einen Rotationsverdampfer). Der Lipidfilm wird mit 9,58 kg 0,9 % NaCl-Lösung versetzt, 3 Stunden lang geschüttelt und unter aseptischen Bedingungen nacheinander durch Membranfilter 0,8 und 0,2 µm filtriert. Die Abfüllung erfolgt aseptisch zu 2 g in Injektionsflaschen der hydrolytischen Klasse I. Die Flaschen werden mit Injektionsstopfen verschlossen.
1 Injektionsflasche enthält 40 mg Wirkstoff in 2 g Phospholipiddispersion.

## Patentansprüche

1. Platin(II oder IV)-Komplexe der allgemeinen Formel

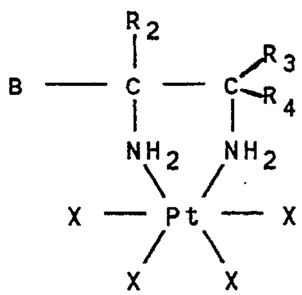

I

worin B einen Phenyl-$C_1$-$C_4$-alkylrest bedeutet, welcher gegebenenfalls im Phenylkern durch den Rest $R_1$ substituiert ist und $R_1$ Wasserstoff, Halogen, Trihalogenmethyl, $C_1$-$C_6$-Alkyl, Hydroxy, $C_1$-$C_6$-Alkoxy oder $C_2$-$C_6$-Alkanoyloxy ist oder worin B zusammen mit dem Strukturteil

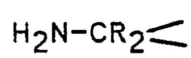

einen Tetrahydroisochinolinrest bildet, falls B Benzyl und $R_2$ Wasserstoff und der Benzylrest in 2-Stellung den $CH_2$-Rest enthält oder worin B zusammen mit dem Strukturteil

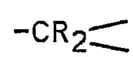

einen Tetrahydronaphthylrest darstellt, bei dem gegebenenfalls eine $CH_2$-Gruppe durch Sauerstoff ersetzt ist, oder worin B zusammen mit dem Strukturteil

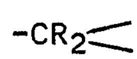

einen Decahydronaphthylrest oder einen Indanylrest darstellt; $R_2$ Wasserstoff, $C_1$-$C_6$-Alkyl, Phenyl oder Phenyl-$C_1$-$C_4$-alkyl bedeutet, wobei der Phenylring dieser Gruppe $R_2$ auch durch Hydroxy, $C_1$-$C_4$-Alkoxy, $C_1$-$C_4$-Alkyl, $C_2$-$C_6$-Alkanoyloxy oder Halogen substituiert sein kann; die Reste $R_3$ und $R_4$ gleich oder verschieden sind und Wasserstoff, $C_1$-$C_{12}$-Alkyl, $C_3$-$C_8$-Cycloalkyl, Phenyl, welches gegebenenfalls durch $C_1$-$C_6$-Alkoxy substituiert ist bedeuten und, falls B ein Benzylrest (gegebenenfalls wie angegeben substituiert) ist, mindestens einer der Reste $R_2$, $R_3$ und $R_4$ kein Wasserstoff ist und X für das Äquivalent eines physiologisch verträglichen Anions steht oder X auch ein Wassermolekül sein kann, wobei im letzteren Falle die fehlende negative Ladung durch ein entsprechendes physiologisch verträgliches Säureanion abgesättigt wird, wobei im Falle von Platin(II)-Komplexen zwei der Gruppen X entfallen.

2. Im Ethylenteil substituierte Phenylalkyl-ethylendiamine der allgemeinen Formel

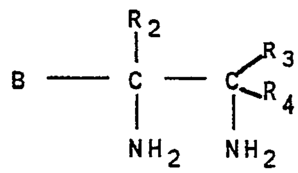

II

worin B einen Phenyl-$C_1$-$C_4$-alkylrest bedeutet, welcher gegebenenfalls im Phenylkern durch den Rest $R_1$ substituiert ist und $R_1$ Wasserstoff, Halogen, Trihalogenmethyl, $C_1$-$C_6$-Alkyl, Hydroxy, $C_1$-$C_6$-Alkoxy oder $C_2$-$C_6$-Alkanoyloxy ist oder worin B zusammen mit dem Strukturteil

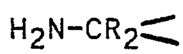

einen Tetrahydroisochinolinrest bildet, falls B Benzyl und $R_2$ Wasserstoff und der Benzylrest in 2-Stellung den $CH_2$-Rest enthält oder worin B zusammen mit dem Strukturteil

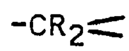

einen Tetrahydronaphthylrest darstellt, bei dem gegebenenfalls eine $CH_2$-Gruppe durch Sauerstoff ersetzt ist, oder worin B zusammen mit dem Strukturteil

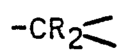

einen Decahydronaphthylrest oder einen Indanylrest darstellt; $R_2$ Wasserstoff, $C_1$-$C_4$-Alkyl, Phenyl oder Phenyl-$C_1$-$C_4$-alkyl bedeutet, wobei der Phenylring dieser Gruppe $R_2$ auch durch Hydroxy, $C_1$-$C_4$-Alkoxy, $C_1$-$C_4$-Alkyl, $C_2$-$C_6$-Alkanoyloxy oder Halogen substituiert sein kann, die Reste $R_3$ und $R_4$ gleich oder verschieden sind und Wasserstoff, $C_1$-$C_{12}$-Alkyl, $C_3$-$C_8$-Cycloalkyl, Phenyl, welches gegebenenfalls durch $C_1$-$C_6$-Alkoxy substituiert ist, bedeuten und, falls B ein Benzylrest (gegebenenfalls wie angegeben substituiert) ist, mindestens einer der Reste $R_2$, $R_3$ und $R_4$ kein Wasserstoff ist und deren Salze.

3. Verfahren zur Herstellung von Platin(II oder IV)-Komplexen der allgemeinen Formel

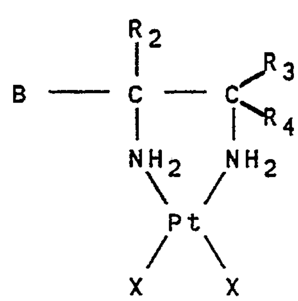

I

worin B einen Phenyl-$C_1$-$C_4$-alkylrest bedeutet, welcher gegebenenfalls im Phenylkern durch den Rest $R_1$ substituiert ist und $R_1$ Wasserstoff, Halogen, Trihalogenmethyl, $C_1$-$C_6$-Alkyl, Hydroxy, $C_1$-$C_6$-Alkoxy oder $C_2$-$C_6$-Alkanoyloxy ist oder worin B zusammen mit dem Strukturteil

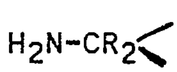

einen Tetrahydroisochinolinrest bildet, falls B Benzyl und $R_2$ Wasserstoff und der Benzylrest in 2-Stellung den $CH_2$-Rest enthält oder worin B zusammen mit dem Strukturteil

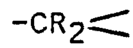

einen Tetrahydronaphthylrest darstellt, bei dem gegebenenfalls eine $CH_2$-Gruppe durch Sauerstoff ersetzt ist, oder worin B zusammen mit dem Strukturteil

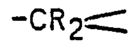

einen Decahydronaphthylrest oder einen Indanylrest darstellt; $R_2$ Wasserstoff, $C_1$-$C_6$-Alkyl, Phenyl oder Phenyl-$C_1$-$C_4$-alkyl bedeutet, wobei der Phenylring dieser Gruppe $R_2$ auch durch Hydroxy, $C_1$-$C_4$-Alkoxy, $C_1$-$C_4$-Alkyl, $C_2$-$C_6$-Alkanoyloxy oder Halogen substituiert sein kann;
die Reste $R_3$ und $R_4$ gleich oder verschieden sind und Wasserstoff, $C_1$-$C_{12}$-Alkyl, $C_3$-$C_8$-Cycloalkyl, Phenyl, welches gegebenenfalls durch $C_1$-$C_6$-Alkoxy substituiert ist, bedeuten und, falls B ein Benzylrest (gegebenenfalls wie angegeben substituiert) ist, mindestens einer der Reste $R_2$, $R_3$ und $R_4$ kein Wasserstoff ist und X für das Äquivalent eines physiologisch verträglichen Anions steht oder X auch ein Wassermolekül sein kann, wobei im letzteren Falle die fehlende negative Ladung durch ein entsprechendes physiologisch verträgliches Säureanion abgesättigt wird, dadurch gekennzeichnet, daß man eine Tetrahalogenoplatin(II)-säure, ein Tetrahalogeno-platin(II)Komplexsalz mit zwei einwertigen oder einem zweiwertigen Kation oder ein Platin(II)-halogenid mit einer Verbindung der Formel

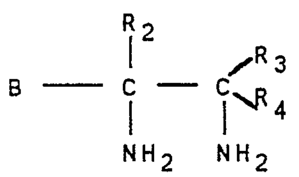

II

oder einem Säureadditionssalz der Verbindung II umsetzt, wobei B, $R_2$, $R_3$ und $R_4$ die zuvor angegebenen Bedeutungen haben, gegebenenfalls zur Überführung in die Platin(IV)-Komplexe die erhaltenen Platin(II)-Komplexe mit oder ohne Anwesenheit einer Verbindung HX oxydiert, und gegebenenfalls in den erhaltenen Platin(II oder IV)-Komplexen der Formel I den Rest X beziehungsweise die Reste X gegen andere physiologisch verträgliche Anionen austauscht und/oder gegebenenfalls die erhaltenen Verbindungen in die Salze mit physiologisch verträglichen Anionen oder Kationen über-führt.

**4.** Verfahren zur Herstellung von im Ethylenteil substituierten Phenylalkyl-ethylendiaminen der allgemeinen Formel

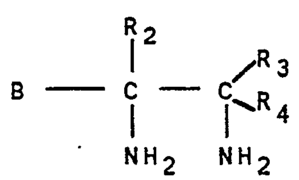

II

worin B einen Phenyl-$C_1$-$C_4$-alkylrest bedeutet, welcher gegebenenfalls im Phenylkern durch den Rest $R_1$ substituiert ist und $R_1$ Wasserstoff, Halogen, Trihalogenmethyl, $C_1$-$C_6$-Alkyl, Hydroxy, $C_1$-$C_6$-Alkoxy oder $C_2$-$C_6$-Alkanoyloxy ist oder worin B zusammen mit dem Strukturteil

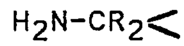

einen Tetrahydroisochinolinrest bildet, falls B Benzyl und $R_2$ Wasserstoff und der Benzylrest in 2-Stellung den $CH_2$-Rest enthält oder worin B zusammen mit dem Strukturteil

$$-CR_2<$$

einen Tetrahydronaphthylrest darstellt, bei dem gegebenenfalls eine $CH_2$-Gruppe durch Sauerstoff ersetzt ist, oder worin B zusammen mit dem Strukturteil

$$-CR_2<$$

einen Decahydronaphthylrest oder einen Indanylrest darstellt; $R_2$ Wasserstoff, $C_1$-$C_6$-Alkyl , Phenyl oder Phenyl - $C_1$-$C_4$-alkyl bedeutet, wobei der Phenylring dieser Gruppe $R_2$ auch durch Hydroxy, $C_1$-$C_4$-Alkoxy, $C_1$-$C_4$-Alkyl, $C_2$-$C_6$-Alkanoyloxy oder Halogen substituiert sein kann;
die Reste $R_3$ und $R_4$ gleich oder verschieden sind und Wasserstoff, $C_1$-$C_{12}$-Alkyl, $C_3$-$C_8$-Cycloalkyl, Phenyl, welches gegebenenfalls durch $C_1$-$C_6$-Alkoxy substituiert ist, bedeuten und, falls B ein Benzylrest (gegebenenfalls wie angegeben substituiert) ist, mindestens einer der Reste $R_2$, $R_3$ und $R_4$ kein Wasserstoff ist und X für das Äquivalent eines physiologisch verträglichen Anions steht oder deren Salzen, dadurch gekennzeichnet, daß man Verbindungen der allgemeinen Formel

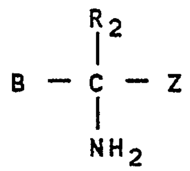

III.

worin Z entweder die Cyangruppe oder die Gruppe $-CONH_2$ ist oder die Gruppe

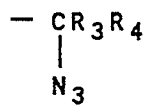

darstellt oder deren Salze zu den entsprechenden Diaminen reduziert, wobei in der Formel III der Rest B einen Phenyl-$C_1$-$C_4$-alkylrest bedeutet, welcher gegebenenfalls im Phenylkern durch den Rest $R_1$ substituiert ist und $R_1$ Wasserstoff, Halogen, Trihalogenmethyl, $C_1$-$C_6$-Alkyl, Hydroxy, $C_1$-$C_6$-Alkoxy oder $C_2$-$C_6$-Alkanoyloxy ist oder worin B zusammen mit dem Strukturteil

$$H_2N-CR_2<$$

einen Tetrahydroisochinolinrest bildet, falls B Benzyl und $R_2$ Wasserstoff und der Benzylrest in 2-Stellung den $CH_2$-Rest enthält oder worin B zusammen mit dem Strukturteil

$$-CR_2<$$

einen Tetrahydronaphthylrest darstellt, bei dem gegebenenfalls eine $CH_2$-Gruppe durch Sauerstoff ersetzt ist, oder worin B zusammen mit dem Strukturteil

$$-CR_2<$$

einen Decahydronaphthylrest oder einen Indanylrest darstellt;
$R_2$ Wasserstoff, $C_1$-$C_6$-Alkyl, Phenyl oder Phenyl-$C_1$-$C_4$-alkyl bedeutet, wobei der Phenylring dieser Gruppe $R_2$ auch durch Hydroxy, $C_1$-$C_4$-Alkoxy, $C_1$-$C_4$-Alkyl, $C_2$-$C_6$-Alkanoyloxy oder Halogen substituiert sein kann;
die Reste $R_3$ und $R_4$ gleich oder verschieden sind und Wasserstoff, $C_1$-$C_{12}$-Alkyl, $C_3$-$C_8$-Cycloalkyl,

Phenyl, welches gegebenenfalls durch $C_1$-$C_6$-Alkoxy substituiert ist, bedeuten und, falls B ein Benzylrest (gegebenenfalls wie angegeben substituiert) ist, mindestens einer der Reste $R_2$, $R_3$ und $R_4$ kein Wasserstoff ist.

**5.** Arzneimittel, enthaltend eine Verbindung der allgemeinen Formel I, neben üblichen Träger-und/oder Verdünnungs- beziehungsweise Hilfsstoffen.

**6.** Verfahren zur Herstellung eines Arzneimittels, dadurch gekennzeichnet, daß eine Verbindung der allgemeinen Formel I mit gebräuchlichen pharmazeutischen Trägerstoffen oder Verdünnungsmitteln beziehungsweise sonstigen Hilfsstoffen zu pharmazeutischen Zubereitungen verarbeitet beziehungsweise in eine therapeutisch anwendbare Form gebracht wird.

**7.** Lösungen, Emulsionen oder Suspensionen von Platin(II oder IV)-Komplexen der Formel I in Wasser oder anderen physiologisch verträglichen Mitteln oder in Mischungen der vorstehend genannten Mittel, dadurch gekennzeichnet, daß diese Lösungsvermittler enthalten.

**8.** Lösungen, Emulsionen oder Suspensionen nach Anspruch 7, dadurch gekennzeichnet, daß die Lösungsvermittler Cyclodextrine oder Polyvinylpyrrolidone sind.

**9.** Verfahren zur Herstellung von Lösungen, Emulsionen oder Suspensionen von Platin(II oder IV)-Komplexen der Formel I in Wasser oder anderen physiologisch verträglichen Mitteln oder in Mischungen der vorstehend genannten Mittel, dadurch gekennzeichnet, daß man Platin(II oder IV)-Komplexe der Formel I und einen Lösungsvermittler in einem üblichen Lösungsmittel/Lösungsmittelgemisch auf eine Temperatur zwischen $40^0$C und $100^0$C erwärmt, gegebenenfalls das Lösungs- beziehungsweise Suspensionsmittel entfernt und in diesem Falle den Rückstand in einem physiologisch verträglichen Mittel auflöst oder suspendiert, beziehungsweise emulgiert.

**10.** Verfahren nach Anspruch 9 dadurch gekennzeichnet, daß als Lösungsvermittler Cyclodextrine oder Polyvinylpyrrolidone verwendet werden.

**11.** Verwendung von Verbindungen der allgemeinen Formel I, zur Herstellung von Arzneimitteln.

Europäisches Patentamt

# EUROPÄISCHER RECHERCHENBERICHT

Nummer der Anmeldung

EP 91 10 5514

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,X | CHEMISCHE BERICHTE. vol. 123, no. 5, Mai 1990, WEINHEIM DE Seiten 1029 - 1038; HENRI BRUNNER ET AL.: "SYNTHESIS AND ANTITUMOR ACTIVITY OF Pt[II]_COMPLEXES OF BENZYL-1,2-DIAMINOETHANE LIGANDS" * das ganze Dokument * | 1-11 | C 07 C 211/27<br>C 07 F 15/00<br>A 61 K 31/28 |
| X | BULLETIN OF THE CHEMICAL SOCIETY OF JAPAN. vol. 56, no. 11, 1983, TOKYO JP Seiten 3268 - 3271; WAKAKO KANDA ET AL.: "SYNTHESIS AND CHARACTERIZATION OF A NEW QUINQUEDENTATE LIGAND CONSISTING OF SALEN SKELETON AND PHENOLIC TAIL CAPABLE OF AXIAL COORDINATION AND ITS METAL COMPLEXES" * Seite 1 * | 2 | |
| X | JOURNAL OF THE AMERICAN CHEMICAL SOCIETY. vol. 109, no. 10, 13 Mai 1987, GASTON, PA US Seiten 3152 - 3154; ERIC J. ROSKAMP ET AL.: "CONVENIENT ROUTES TO VICINAL DIAMINES. COUPLING OF NITRILES OR N-[TRIMETHYLSILYL]IMINES PROMOTED BY NbCL4[THF]2" * Seite 3153 * | 2 | |
| | | | RECHERCHIERTE SACHGEBIETE (Int. Cl.5) |
| A,D | EP-A-0 193 083 (ASTA-WERKE AG) * das ganze Dokument & DE-A-3605191 * | 1-7 | C 07 F<br>C 07 C<br>A 61 K |
| A | EUROPEAN JOURNAL OF MEDICINAL CHEMISTRY. vol. 25, no. 1, Januar 1990, ELSEVIER PARIS FR. Seiten 35 - 44; HENRI BRUNNER ET AL.: "SYNTHESIS AND ANTITUMOR ACTIVITY OF PLATINUM[II] COMPLEXES CONTAINING SUBSTITUTED ETHYLENEDIAMINE LIGANDS" * das ganze Dokument * | 1-7 | |

-/-

Der vorliegende Recherchenbericht wurde für alle Patentansprüche erstellt

| Recherchenort | Abschlußdatum der Recherche | Prüfer |
|---|---|---|
| Berlin | 11 Juni 91 | RUFET J.M.A. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument

Europäisches Patentamt

# EUROPÄISCHER RECHERCHENBERICHT

Nummer der Anmeldung

**EP 91 10 5514**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | CHEMICAL ABSTRACTS, vol. 109, no. 21, 21 November 1988 Columbus, Ohio, USA GEORGIADIS MINAS P. ET AL.: "PRODUCTS FROM FURANS. V. SYNTHESIS OF OXYGEN-CONTAINING ISOSTERES OF SYMPATHOMIMETIC AMINES VIA 6-HYDROXY-2H-PYRAN-3[6H]-ONES AND ....."<br>& J. HETEROCYCL. CHEM. 1988 BD 25[3] SEITEN 995-1002 EP 91105514030Seite 690; rechte Spalte; ref. no. 190186E<br>* Zusammenfassung *<br>----- | 1 | |

| | RECHERCHIERTE SACHGEBIETE (Int. Cl.5) |
|---|---|
| | |

Der vorliegende Recherchenbericht wurde für alle Patentansprüche erstellt

| Recherchenort | Abschlußdatum der Recherche | Prüfer |
|---|---|---|
| Berlin | 11 Juni 91 | RUFET J.M.A. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument